# EUROPEAN PATENT APPLICATION

(11) **EP 1 897 953 A1**
(43) Date of publication of application: **12.03.2008**
(21) Application number: 06018713.5
(22) Date of filing: 06.09.2006
(51) Int. Cl.: C12N 15/82, C12N 15/74

(54) **Potexvirus-derived replicon**

(71) Applicant: Icon Genetics GmbH, 80333 München (DE)
(72) Inventor: Marillonnet, Sylvestre, 06126 Halle (DE); Engler, Carola, 06120 Halle (DE); Klimyuk, Victor, 06114 Halle (DE); Gleba, Yuri, 06114 Halle (DE)
(74) Representative: Blodig, Wolfgang

(57) **Abstract**

Nucleic acid comprising or encoding an RNA replicon comprising, in this order, the following segments:
(i) a nucleic acid sequence encoding a potexvirus RNA-dependent RNA polymerase or a function-conservative variant thereof;
(ii) a nucleic acid sequence encoding one or more proteins required for cell-to-cell movement of said replicon in a plant or in plant tissue;
(iii) a heterologous nucleic acid sequence expressible from said replicon in a plant or in plant tissue.

SEQUENCE LISTING

<110>  Icon Genetics AG

<120>  Potexvirus-derived replicon

<130>  EAD-14138

<160>  6

<170>  PatentIn version 3.1

<210>  1

<211>  786

<212>  DNA

<213>  Artificial Sequence

<220>

<223>  Fragment of pICH22939 (subgenomic promoter-TMV MP, between 25K an
d subgenomic promoter-gene of interest)

<400>  1
tactcgaaag aggtcagcac cagctagaaa atgtcgatag tctcgtacga acctaaggtg      60

agtgatttcc tcaatctttc gaagaaggaa gagatcttgc cgaaggctct aacgaggtta     120

aaaaccgtgt ctattagtac taaagatatt atatctgtca aggagtcgga gactttgtgt     180

gatatagatt tgttaatcaa tgtgccatta gataagtata gatatgtggg tatcctagga     240

gctgttttta ccggagagtg gctagtgcca gacttcgtta aaggtggagt gacgataagt     300

gtgatagata agcgtctggt gaactcaaag gagtgcgtga ttggtacgta cagagccgca     360

gccaagagta agaggttcca gttcaaattg gttccaaatt actttgtgtc caccgtggac     420

gcaaagagga agccgtggca ggttcatgtt cgtatacaag acttgaagat tgaggcgggt     480

tggcagccgt tagctctgga agtagtttca gttgctatgg tcaccaataa cgttgtcatg     540

aagggtttga gggaaaaggt cgtcgcaata aatgatccgg acgtcgaagg tttcgaaggt     600

gtggttgacg aattcgtcga ttcggttgca gcatttaaag cggttgacaa tttcaggaag     660

aggaaaaaga aggttgaaga aagggatgta gtaagtaagt ataaatatag accggagaaa     720

tacgccggtc ctgattcgtt taatttaaaa gaagaaaatg tcttacaaca ttacaaaccc     780

gaataa                                                                786

<210> 2

<211> 792

<212> DNA

<213> Artificial Sequence

<220>

<223> Sequence fragment from pICH22953 containing the 11 C-terminal ami
      noacids of the 25K coding sequence, followed by the TVCV MP codin
      g sequence (the ATG of the 12K protein in the 25K sequence is mut
      ated from ATG to ACG)

<400> 2
caaagggatt gacatacgtc cgcgcagggc catagaaaaa tgtcgatagt ctcgtacgaa      60

cctaaggtga gtgatttcct caatctttcg aagaaggaag agatcttgcc gaaggctcta     120

acgaggttaa aaaccgtgtc tattagtact aaagatatta tatctgtcaa ggagtcggag     180

actttgtgtg atatagattt gttaatcaat gtgccattag ataagtatag atatgtgggt     240

atcctaggag ctgtttttac cggagagtgg ctagtgccag acttcgttaa aggtggagtg     300

acgataagtg tgatagataa gcgtctggtg aactcaaagg agtgcgtgat tggtacgtac     360

agagccgcag ccaagagtaa gaggttccag ttcaaattgg ttccaaatta ctttgtgtcc     420

accgtggacg caaagaggaa gccgtggcag gttcatgttc gtatacaaga cttgaagatt     480

gaggcgggtt ggcagccgtt agctctggaa gtagtttcag ttgctatggt caccaataac     540

gttgtcatga agggtttgag ggaaaaggtc gtcgcaataa atgatccgga cgtcgaaggt     600

ttcgaaggtg tggttgacga attcgtcgat tcggttgcag catttaaagc ggttgacaat     660

ttcaggaaga ggaaaaagaa ggttgaagaa agggatgtag taagtaagta taaatataga     720

ccggagaaat acgccggtcc tgattcgttt aatttaaaag aagaaaatgt cttacaacat     780

tacaaacccg aa                                                         792

<210> 3

<211> 817

<212> DNA

<213> Artificial Sequence

<220>

<223> Sequence fragment of pICH24180 including the 4 last amino acids o

f the RdRP, the subgenomic promoter of the 25K gene, the PVX CP g
ene, the subgenomic promoter of the 25K gene and the first 4 aa o
f the 25K protein

<400> 3
agaaactttc tttaaccgtt aagttacctt agagatttga ataagatgtc agcaccagct 60

agcacaacac agcccatagg gtcaactacc tcaactacca caaaaactgc aggcgcaact 120

cctgccacag cttcaggcct gttcactatc ccggatgggg atttctttag tacagcccgt 180

gccatagtag ccagcaatgc tgtcgcaaca aatgaggacc tcagcaagat tgaggctatt 240

tggaaggaca tgaaggtgcc cacagacact atggcacagg ctgcttggga cttagtcaga 300

cactgtgctg atgtaggatc atccgctcaa acagaaatga tagatacagg tccctattcc 360

aacggcatca gcagagctag actggcagca gcaattaaag aggtgtgcac acttaggcaa 420

ttttgcatga agtatgcccc agtggtatgg aactggatgt taactaacaa cagtccacct 480

gctaactggc aagcacaagg tttcaagcct gagcacaaat tcgctgcatt cgacttcttc 540

aatggagtca ccaacccagc tgccatcatg cccaaagagg ggctcatccg gccaccgtct 600

gaagctgaaa tgaatgctgc ccaaactgct gcctttgtga agattacaaa ggccagggca 660

caatccaacg actttgccag cctagatgca gctgtcactc gaggtcgtat cactggaaca 720

acaaccgctg aggctgttgt cactctacca ccaccataac agaaactttc tttaaccgtt 780

aagttacctt agagatttga ataagatgga tattctc 817

<210> 4

<211> 4371

<212> DNA

<213> Artificial Sequence

<220>

<223> Sequence fragment of pICH25488 encoding RNA-dependent RNA polymer
ase

<400> 4
atggccaagg tgcgcgaggt ttaccaatct tttacagact ccaccacaaa aactctcatc 60

caagatgagg cttatagaaa cattcgcccc atcatggaaa aacacaaact agctaaccct 120

tacgctcaaa cggttgaagc ggctaatgat ctagaggggt tcggcatagc caccaatccc 180

tatagcattg aattgcatac acatgcagcc gctaagacca tagagaataa acttctagag 240

gtgcttggtt ccatcctacc acaagaacct gttacattta tgtttcttaa acccagaaag 300

ctaaactaca tgagaagaaa cccgcggatc aaggacattt tccaaaatgt tgccattgaa 360

ccaagagacg tagccaggta ccccaaggaa acaataattg acaaactcac agagatcaca 420

acggaaacag catacattag tgacactctg cacttcttgg atccgagcta catagtggag 480

acattccaaa actgcccaaa attgcaaaca ttgtatgcga ccttagttct ccccgttgag 540

gcagccttta aaatggaaag cactcacccg aacatataca gcctcaaata cttcggagat 600

ggtttccagt atataccagg caaccatggt ggcggggcat accatcatga attcgctcat 660

ctacaatggc tcaaagtggg aaagatcaag tggagggacc ccaaggatag ctttctcgga 720

catctcaatt acacgactga gcaggttgag atgcacacag tgacagtaca gttgcaggaa 780

tcgttcgcgg caaaccactt gtactgcatc aggagaggag acttgctcac accggaggtg 840

cgcactttcg gccaacctga caggtacgtg attccaccac agatcttcct cccaaaagtt 900

cacaactgca agaagccgat tctcaagaaa actatgatgc agctcttctt gtatgttagg 960

acagtcaagg tcgcaaaaaa ttgtgacatt tttgccaaag tcagacaatt aattaaatca 1020

tctgacttgg acaaatactc tgctgtggaa ctggtttact tagtaagcta catggagttc 1080

cttgccgatt tacaagctac cacctgcttc tcagacacac tttctggtgg cttgctaaca 1140

aagacccttg caccggtgag ggcttggata caagagaaaa agatgcagct gtttggtctt 1200

gaggactacg cgaagttagt caaagcagtt gatttccacc cggtggattt ttctttcaaa 1260

gtggaaactt gggacttcag attccacccc ttgcaagcgt ggaaagcctt ccgaccaagg 1320

gaagtgtcgg atgtagagga aatggaaagt ttgttctcag atggggacct gcttgattgc 1380

ttcacaagaa tgccagctta tgcggtaaac gcagaggaag atttagctgc aatcaggaaa 1440

acgcccgaga tggatgtcgg tcaagaagtt aaagagcctg caggagacag aaatcaatac 1500

tcaaaccctg cagaaacttt cctcaacaag ctccacagga aacacagtag ggaggtgaaa 1560

caccaggccg caaagaaagc taaacgccta gctgaaatcc aggagtcaat gagagctgaa 1620

ggtgatgccg aaccaaatga aataagcggg acgatggggg caatacccag caacgccgaa 1680

cttcctggca cgaatgatgc cagacaagaa ctcacactcc caaccactaa acctgtccct 1740

gcaaggtggg aagatgcttc attcacagat tctagtgtgg aagaggagca ggttaaactc 1800

cttggaaaag aaaccgttga aacagcgacg caacaagtca tcgaaggact tccttggaaa 1860

cactggattc ctcaattaaa tgctgttgga ttcaaggcgc tggaaattca gagggatagg 1920

agtggaacaa tgatcatgcc catcacagaa atggtgtccg ggctggaaaa agaggacttc 1980

cctgaaggaa ctccaaaaga gttggcacga gaattgttcg ctatgaacag aagccctgcc 2040

accatccctt tggacctgct tagagccaga gactacggca gtgatgtaaa gaacaagaga 2100

attggtgcca tcacaaagac acaggcaacg agttggggcg aatacttgac aggaaagata 2160

gaaagcttaa ctgagaggaa agttgcgact tgtgtcattc atggagctgg aggttctgga 2220

aaaagtcatg ccatccagaa ggcattgaga gaaattggca agggctcgga catcactgta 2280

gtcctgccga ccaatgaact gcggctagat tggagtaaga aagtgcctaa cactgagccc 2340

tatatgttca agacctctga aaaggcgtta attgggggaa caggcagcat agtcatcttt 2400

gacgattact caaaacttcc tcccggttac atagaagcct tagtctgttt ctactctaaa 2460

atcaagctaa tcattctaac aggagatagc agacaaagcg tctaccatga aactgctgag 2520

gacgcctcca tcaggcattt gggaccagca acagagtact tctcaaaata ctgccgatac 2580

tatctcaatg ccacacaccg caacaagaaa gatcttgcga acatgcttgg tgtctacagt 2640

gagagaacgg gagtcaccga aatcagcatg agcgccgagt tcttagaagg aatcccaact 2700

ttggtaccct cggatgagaa gagaaagctg tacatgggca ccgggaggaa tgacacgttc 2760

acatacgctg gatgccaggg gctaactaag ccgaaggtac aaatagtgtt ggaccacaac 2820

acccaagtgt gtagcgcgaa tgtgatgtac acggcacttt ctagagccac cgataggatt 2880

cacttcgtga acacaagtgc aaattcctct gccttctggg aaaagttgga cagcacccct 2940

tacctcaaga ctttcctatc agtggtgaga gaacaagcac tcagggagta cgagccggca 3000

gaggcagagc caattcaaga gcctgagccc cagacacaca tgtgtgtcga gaatgaggag 3060

tccgtgctag aagagtacaa agaggaactc ttggaaaagt ttgacagaga gatccactct 3120

gaatcccatg gtcattcaaa ctgtgtccaa actgaagaca caaccattca gttgttttcg 3180

catcaacaag caaaagatga gactctcctc tgggcgacta tagatgcgcg gctcaagacc 3240

agcaatcaag aaacaaactt ccgagaattc ctgagcaaga aggacattgg ggacgttctg 3300

tttttaaact accaaaaagc tatgggttta cccaaagagc gtattccttt ttcccaagag 3360

gtctgggaag cttgtgccca cgaagtacaa agcaagtacc tcagcaagtc aaagtgcaac 3420

ttgatcaatg ggactgtgag acagagccca gacttcgatg aaaataagat tatggtattc 3480

ctcaagtcgc agtgggtcac aaaggtggaa aaactaggtc tacccaagat taagccaggt 3540

caaaccatag cagcctttta ccagcagact gtgatgcttt ttggaactat ggctaggtac 3600

atgcgatggt tcagacaggc tttccagcca aaagaagtct tcataaactg tgagacgacg 3660

ccagatgaca tgtctgcatg ggccttgaac aactggaatt tcagcagacc tagcttggct 3720

aatgactaca cagctttcga ccagtctcag gatggagcca tgttgcaatt tgaggtgctc 3780

aaagccaaac accactgcat accagaggaa atcattcagg catacataga tattaagact 3840

aatgcacaga ttttcctagg cacgttatca attatgcgcc tgactggtga aggtcccact 3900

tttgatgcaa acactgagtg caacatagct tacacccata caaagtttga catcccagcc 3960

ggaactgctc aagtttatgc aggagacgac tccgcactgg actgtgttcc agaagtgaag 4020

catagtttcc acaggcttga ggacaaatta ctcctaaagt caaagcctgt aatcacgcag 4080

caaaagaagg gcagttggcc tgagttttgt ggttggctga tcacaccaaa aggggtgatg 4140

aaagacccaa ttaagctcca tgttagctta aaattggctg aagctaaggg tgaactcaag 4200

aaatgtcaag attcctatga aattgatctg agttatgcct atgaccacaa ggactctctg 4260

catgacttgt tcgatgagaa acagtgtcag gcacacacac tcacttgcag aacactaatc 4320

aagtcaggga gaggcactgt ctcactttcc cgcctcagaa actttcttta a 4371

<210> 5

<211> 8070

<212> DNA

<213> Artificial Sequence

<220>

<223> T-DNA region of plasmid pICH25488

<400> 5
cctgatctgg ggaaccctgt ggttggcaca tacaaatgga cgaacggata aacctttca 60

cgccctttta aatatccgat tattctaata aacgctcttt tctcttaggt ttacccgcca 120

atatatcctg tcaaacactg atagtttaaa ctgaaggcgg gaaacgacaa tctgatctaa 180

gctaggcatg cctgcaggtc aacatggtgg agcacgacac gcttgtctac tccaaaaata 240

tcaaagatac agtctcagaa gaccaaaggg caattgagac ttttcaacaa agggtaatat 300

ccggaaacct cctcggattc cattgcccag ctatctgtca ctttattgtg aagatagtgg 360

aaaaggaagg tggctcctac aaatgccatc attgcgataa aggaaaggcc atcgttgaag 420

atgcctctgc cgacagtggt cccaaagatg gacccccacc cacgaggagc atcgtggaaa 480

aagaagacgt tccaaccacg tcttcaaagc aagtggattg atgtgatatc tccactgacg 540

taagggatga cgcacaatcc cactatcctt cgcaagaccc ttcctctata taaggaagtt 600

catttcattt ggagaggaga aaactaaacc atacaccacc aacacaacca aacccaccac 660

gcccaattgt tacacacccg cttgaaaaag aaagtttaac aaatggccaa ggtgcgcgag 720

gtttaccaat cttttacaga ctccaccaca aaaactctca tccaagatga ggcttataga 780

aacattcgcc ccatcatgga aaaacacaaa ctagctaacc cttacgctca aacggttgaa 840

gcggctaatg atctagaggg gttcggcata gccaccaatc cctatagcat tgaattgcat 900

acacatgcag ccgctaagac catagagaat aaacttctag aggtgcttgg ttccatccta 960

ccacaagaac ctgttacatt tatgtttctt aaacccagaa agctaaacta catgagaaga 1020

aacccgcgga tcaaggacat tttccaaaat gttgccattg aaccaagaga cgtagccagg 1080

taccccaagg aaacaataat tgacaaactc acagagatca caacggaaac agcatacatt 1140

agtgacactc tgcacttctt ggatccgagc tacatagtgg agacattcca aaactgccca 1200

aaattgcaaa cattgtatgc gaccttagtt ctccccgttg aggcagcctt taaaatggaa 1260

agcactcacc cgaacatata cagcctcaaa tacttcggag atggtttcca gtatatacca 1320

ggcaaccatg gtggcggggc ataccatcat gaattcgctc atctacaatg gctcaaagtg 1380

ggaaagatca agtggaggga ccccaaggat agctttctcg gacatctcaa ttacacgact 1440

gagcaggttg agatgcacac agtgacagta cagttgcagg aatcgttcgc ggcaaaccac 1500

ttgtactgca tcaggagagg agacttgctc acaccggagg tgcgcacttt cggccaacct 1560

gacaggtacg tgattccacc acagatcttc ctcccaaaag ttcacaactg caagaagccg 1620

attctcaaga aaactatgat gcagctcttc ttgtatgtta ggacagtcaa ggtcgcaaaa 1680

aattgtgaca tttttgccaa agtcagacaa ttaattaaat catctgactt ggacaaatac 1740

tctgctgtgg aactggttta cttagtaagc tacatggagt tccttgccga tttacaagct 1800

accacctgct tctcagacac actttctggt ggcttgctaa caaagaccct tgcaccggtg 1860

agggcttgga tacaagagaa aaagatgcag ctgtttggtc ttgaggacta cgcgaagtta 1920

gtcaaagcag ttgatttcca cccggtggat ttttctttca aagtggaaac ttgggacttc 1980

agattccacc ccttgcaagc gtggaaagcc ttccgaccaa gggaagtgtc ggatgtagag 2040

gaaatggaaa gtttgttctc agatggggac ctgcttgatt gcttcacaag aatgccagct 2100

tatgcggtaa acgcagagga agatttagct gcaatcagga aaacgcccga gatggatgtc 2160

ggtcaagaag ttaaagagcc tgcaggagac agaaatcaat actcaaaccc tgcagaaact 2220

ttcctcaaca agctccacag gaaacacagt agggaggtga aacaccaggc cgcaaagaaa 2280

gctaaacgcc tagctgaaat ccaggagtca atgagagctg aaggtgatgc cgaaccaaat 2340

gaaataagcg ggacgatggg ggcaataccc agcaacgccg aacttcctgg cacgaatgat 2400

gccagacaag aactcacact cccaaccact aaacctgtcc ctgcaaggtg ggaagatgct 2460

tcattcacag attctagtgt ggaagaggag caggttaaac tccttggaaa agaaaccgtt 2520

gaaacagcga cgcaacaagt catcgaagga cttccttgga aacactggat tcctcaatta 2580

aatgctgttg gattcaaggc gctggaaatt cagagggata ggagtggaac aatgatcatg 2640

cccatcacag aaatggtgtc cgggctggaa aaagaggact tccctgaagg aactccaaaa 2700

gagttggcac gagaattgtt cgctatgaac agaagccctg ccaccatccc tttggacctg 2760

cttagagcca gagactacgg cagtgatgta aagaacaaga gaattggtgc catcacaaag 2820

acacaggcaa cgagttgggg cgaatacttg acaggaaaga tagaaagctt aactgagagg 2880

aaagttgcga cttgtgtcat tcatggagct ggaggttctg gaaaaagtca tgccatccag 2940

aaggcattga gagaaattgg caagggctcg gacatcactg tagtcctgcc gaccaatgaa 3000

ctgcggctag attggagtaa gaaagtgcct aacactgagc cctatatgtt caagacctct 3060

gaaaaggcgt taattggggg aacaggcagc atagtcatct ttgacgatta ctcaaaactt 3120

cctcccggtt acatagaagc cttagtctgt ttctactcta aaatcaagct aatcattcta 3180

acaggagata gcagacaaag cgtctaccat gaaactgctg aggacgcctc catcaggcat 3240

ttgggaccag caacagagta cttctcaaaa tactgccgat actatctcaa tgccacacac 3300

cgcaacaaga aagatcttgc gaacatgctt ggtgtctaca gtgagagaac gggagtcacc 3360

gaaatcagca tgagcgccga gttcttagaa ggaatcccaa ctttggtacc ctcggatgag 3420

aagagaaagc tgtacatggg caccgggagg aatgacacgt tcacatacgc tggatgccag 3480

gggctaacta agccgaaggt acaaatagtg ttggaccaca acacccaagt gtgtagcgcg 3540

aatgtgatgt acacggcact ttctagagcc accgatagga ttcacttcgt gaacacaagt 3600

gcaaattcct ctgccttctg ggaaaagttg gacagcaccc cttacctcaa gactttccta 3660

tcagtggtga gagaacaagc actcagggag tacgagccgg cagaggcaga gccaattcaa 3720

gagcctgagc cccagacaca catgtgtgtc gagaatgagg agtccgtgct agaagagtac 3780

aaagaggaac tcttggaaaa gtttgacaga gagatccact ctgaatccca tggtcattca 3840

aactgtgtcc aaactgaaga cacaaccatt cagttgtttt cgcatcaaca agcaaaagat 3900

gagactctcc tctgggcgac tatagatgcg cggctcaaga ccagcaatca agaaacaaac 3960

ttccgagaat tcctgagcaa gaaggacatt ggggacgttc tgtttttaaa ctaccaaaaa 4020

gctatgggtt tacccaaaga gcgtattcct ttttcccaag aggtctggga agcttgtgcc 4080

cacgaagtac aaagcaagta cctcagcaag tcaaagtgca acttgatcaa tgggactgtg 4140

agacagagcc cagacttcga tgaaaataag attatggtat tcctcaagtc gcagtgggtc 4200

acaaaggtgg aaaaactagg tctacccaag attaagccag gtcaaaccat agcagccttt 4260

taccagcaga ctgtgatgct ttttggaact atggctaggt acatgcgatg gttcagacag 4320

gctttccagc caaaagaagt cttcataaac tgtgagacga cgccagatga catgtctgca 4380

tgggccttga acaactggaa tttcagcaga cctagcttgg ctaatgacta cacagctttc 4440
gaccagtctc aggatggagc catgttgcaa tttgaggtgc tcaaagccaa acaccactgc 4500
ataccagagg aaatcattca ggcatacata gatattaaga ctaatgcaca gattttccta 4560
ggcacgttat caattatgcg cctgactggt gaaggtccca cttttgatgc aaacactgag 4620
tgcaacatag cttacaccca tacaaagttt gacatcccag ccggaactgc tcaagtttat 4680
gcaggagacg actccgcact ggactgtgtt ccagaagtga agcatagttt ccacaggctt 4740
gaggacaaat tactcctaaa gtcaaagcct gtaatcacgc agcaaaagaa gggcagttgg 4800
cctgagtttt gtggttggct gatcacacca aaaggggtga tgaaagaccc aattaagctc 4860
catgttagct taaaattggc tgaagctaag ggtgaactca agaaatgtca agattcctat 4920
gaaattgatc tgagttatgc ctatgaccac aaggactctc tgcatgactt gttcgatgag 4980
aaacagtgtc aggcacacac actcacttgc agaacactaa tcaagtcagg gagaggcact 5040
gtctcacttt cccgcctcag aaactttctt taaccgttaa gttaccttag agatttgaat 5100
aagatggata ttctcatcag tagtttgaaa agtttaggtt attctaggac ttccaaatct 5160
ttagattcag gacctttggt agtacatgca gtagccggag ccggtaagtc cacagcccta 5220
aggaagttga tcctcagaca cccaacattc accgtgcata cactcggtgt ccctgacaag 5280
gtgagtatca gaactagagg catacagaag ccaggaccta ttcctgaggg caacttcgca 5340

atcctcgatg agtatacttt ggacaacacc acaaggaact cataccaggc actttttgct 5400
gacccttatc aggcaccgga gtttagccta gagccccact tctacttgga aacatcattt 5460
cgagttccga ggaaagtggc agatttgata gctggctgtg gcttcgattt cgagacgaac 5520
tcaccggaag aagggcactt agagatcact ggcatattca aagggcccct actcggaaag 5580
gtgatagcca ttgatgagga gtctgagaca acactgtcca ggcatggtgt tgagtttgtt 5640
aagccctgcc aagtgacggg acttgagttc aaagtagtca ctattgtgtc tgccgcacca 5700
atagaggaaa ttggccagtc cacagctttc tacaacgcta tcaccaggtc aaagggattg 5760
acatatgtcc gcgcagggcc ataggctgac cgctccggtc aattctgaaa aagtgtacat 5820
agtattaggt ctatcatttg ctttagtttc aattaccttt ctgctttcta gaaatagctt 5880
accccacgtc ggtgacaaca ttcacagctt gccacacgga ggagcttaca gagacggcac 5940
caaagcaatc ttgtacaact ccccaaatct agggtcacga gtgagtctac acaacggaaa 6000
gaacgcagca tttgctgccg ttttgctact gactttgctg atctatggaa gtaaatacat 6060
atctcaacgc aatcatactt gtgcttgtgg taacaatcat agcagtcatt agcacttcct 6120
tagtgaggac tgaaccttgt gtcatcaaga ttactgggga atcaatcaca gtgttggctt 6180

gcaaactaga tgcagaaacc ataagggcca ttgccgatct caagccactc tccgttgaac 6240

ggttaagttt ccattgatac tcgaaagatg tcagcaccag ctagtacaac acagcccata 6300

gggtcaacta cctcaactac cacaaaaact gcaggcgcaa ctcctgccac agcttcaggc 6360

ctgttcacta tcccggatgg ggatttcttt agtacagccc gtgccatagt agccagcaat 6420

gctgtcgcaa caaatgagga cctcagcaag attgaggcta tttggaagga catgaaggtg 6480

cccacagaca ctatggcaca ggctgcttgg gacttagtca gacactgtgc tgatgtagga 6540

tcatccgctc aaacagaaat gatagataca ggtccctatt ccaacggcat cagcagagct 6600

agactggcag cagcaattaa agaggtgtgc acacttaggc aattttgcat gaagtatgcc 6660

ccagtggtat ggaactggat gttaactaac aacagtccac ctgctaactg gcaagcacaa 6720

ggtttcaagc ctgagcacaa attcgctgca ttcgacttct tcaatggagt caccaaccca 6780

gctgccatca tgcccaaaga ggggctcatc cggccaccgt ctgaagctga aatgaatgct 6840

gcccaaactg ctgcctttgt gaagattaca aaggccaggg cacaatccaa cgactttgcc 6900

agcctagatg cagctgtcac tcgaggtcgt atcactggaa caacaaccgc tgaggctgtt 6960

gtcactctac caccaccata atgaacggtt aagtttccat tgatactcga aagaggtcag 7020

caccagctag caacaaacaa gaaatggtga gcaagggcga ggagctgttc accggggtgg 7080

tgcccatcct ggtcgagctg gacggcgacg taaacggcca caagttcagc gtgtccggcg 7140

agggcgaggg cgatgccacc tacggcaagc tgaccctgaa gttcatctgc accaccggca 7200

agctgcccgt gccctggccc accctcgtga ccaccttcag ctacggcgtg cagtgcttca 7260

gccgctaccc cgaccacatg aagcagcacg acttcttcaa gtccgccatg cccgaaggct 7320

acgtccagga gcgcaccatc ttcttcaagg acgacggcaa ctacaagacc cgcgccgagg 7380

tgaagttcga gggcgacacc ctggtgaacc gcatcgagct gaagggcatc gacttcaagg 7440

aggacggcaa catcctgggg cacaagctgg agtacaacta caacagccac aacgtctata 7500

tcatggccga caagcagaag aacggcatca aggtgaactt caagatccgc cacaacatcg 7560

aggacggcag cgtgcagctc gccgaccact accagcagaa cacccccatc ggcgacggcc 7620

ccgtgctgct gcccgacaac cactacctga gcacccagtc cgccctgagc aaagacccca 7680

acgagaagcg cgatcacatg gtcctgctgg agttcgtgac cgccgccggg atcactcacg 7740

gcatggacga gctgtacaag taatctagcg ataccgtcga ctacgtctac ataaccgacg 7800

cctaccccag tttcatagta ttttctggtt tgattgtatg aataatataa ataaaaaaaa 7860

aaaaaaaaaa aaaaaactag tggtaccgag ctcttctgtc agcgggccca ctgcatccac 7920

cccagtacat taaaaacgtc cgcaatgtgt tattaagttg tctaagcgtc aatttgttta 7980

caccacaata tatcctgcca ccagccagcc aacagctccc cgaccggcag ctcggcacaa 8040

aatcaccact cgatacaggc agcccatcag 8070

<210> 6

<211> 8060

<212> DNA

<213> Artificial Sequence

<220>

<223> T-DNA region of plasmid pICH25491

<400> 6
cctgtggttg gcacatacaa atggacgaac ggataaacct tttcacgccc ttttaaatat 60

ccgattattc taataaacgc tcttttctct taggtttacc cgccaatata tcctgtcaaa 120

cactgatagt ttaaactgaa ggcgggaaac gacaatctga tctaagctag gcatgcctgc 180

aggtcaacat ggtggagcac gacacgcttg tctactccaa aaatatcaaa gatacagtct 240

cagaagacca aagggcaatt gagacttttc aacaaagggt aatatccgga aacctcctcg 300

gattccattg cccagctatc tgtcacttta ttgtgaagat agtggaaaag gaaggtggct 360

cctacaaatg ccatcattgc gataaaggaa aggccatcgt tgaagatgcc tctgccgaca 420

gtggtcccaa agatggaccc ccacccacga ggagcatcgt ggaaaaagaa gacgttccaa 480

ccacgtcttc aaagcaagtg gattgatgtg atatctccac tgacgtaagg gatgacgcac 540

aatcccacta tccttcgcaa gacccttcct ctatataagg aagttcattt catttggaga 600

ggagaaaact aaaccataca ccaccaacac aaccaaaccc accacgccca attgttacac 660

acccgcttga aaaagaaagt ttaacaaatg gccaaggtgc gcgaggttta ccaatctttt 720

acagactcca ccacaaaaac tctcatccaa gatgaggctt atagaaacat tcgccccatc 780

atggaaaaac acaaactagc taacccttac gctcaaacgg ttgaagcggc taatgatcta 840

gaggggttcg gcatagccac caatccctat agcattgaat tgcatacaca tgcagccgct 900

aagaccatag agaataaact tctagaggtg cttggttcca tcctaccaca agaacctgtt 960

acatttatgt ttcttaaacc cagaaagcta aactacatga gaagaaaccc gcggatcaag 1020

gacattttcc aaaatgttgc cattgaacca agagacgtag ccaggtaccc caaggaaaca 1080

ataattgaca aactcacaga gatcacaacg gaaacagcat acattagtga cactctgcac 1140

ttcttggatc cgagctacat agtggagaca ttccaaaact gcccaaaatt gcaaacattg 1200

tatgcgacct tagttctccc cgttgaggca gcctttaaaa tggaaagcac tcacccgaac 1260

atatacagcc tcaaatactt cggagatggt ttccagtata taccaggcaa ccatggtggc 1320

ggggcatacc atcatgaatt cgctcatcta caatggctca aagtgggaaa gatcaagtgg 1380

agggacccca aggatagctt tctcggacat ctcaattaca cgactgagca ggttgagatg 1440

cacacagtga cagtacagtt gcaggaatcg ttcgcggcaa accacttgta ctgcatcagg 1500

agaggagact tgctcacacc ggaggtgcgc actttcggcc aacctgacag gtacgtgatt 1560

ccaccacaga tcttcctccc aaaagttcac aactgcaaga agccgattct caagaaaact 1620

atgatgcagc tcttcttgta tgttaggaca gtcaaggtcg caaaaaattg tgacattttt 1680

gccaaagtca gacaattaat taaatcatct gacttggaca aatactctgc tgtggaactg 1740

gtttacttag taagctacat ggagttcctt gccgatttac aagctaccac ctgcttctca 1800

gacacacttt ctggtggctt gctaacaaag acccttgcac cggtgagggc ttggatacaa 1860

gagaaaaaga tgcagctgtt tggtcttgag gactacgcga agttagtcaa agcagttgat 1920

ttccacccgg tggatttttc tttcaaagtg gaaacttggg acttcagatt ccaccccttg 1980

caagcgtgga aagccttccg accaagggaa gtgtcggatg tagaggaaat ggaaagtttg 2040

ttctcagatg gggacctgct tgattgcttc acaagaatgc cagcttatgc ggtaaacgca 2100

gaggaagatt tagctgcaat caggaaaacg cccgagatgg atgtcggtca agaagttaaa 2160

gagcctgcag gagacagaaa tcaatactca aaccctgcag aaactttcct caacaagctc 2220

cacaggaaac acagtaggga ggtgaaacac caggccgcaa agaaagctaa acgcctagct 2280

gaaatccagg agtcaatgag agctgaaggt gatgccgaac caaatgaaat aagcgggacg 2340

atgggggcaa tacccagcaa cgccgaactt cctggcacga atgatgccag acaagaactc 2400

acactcccaa ccactaaacc tgtccctgca aggtgggaag atgcttcatt cacagattct 2460

agtgtggaag aggagcaggt taaactcctt ggaaaagaaa ccgttgaaac agcgacgcaa 2520

caagtcatcg aaggacttcc ttggaaacac tggattcctc aattaaatgc tgttggattc 2580

aaggcgctgg aaattcagag ggataggagt ggaacaatga tcatgcccat cacagaaatg 2640

gtgtccgggc tggaaaaaga ggacttccct gaaggaactc caaaagagtt ggcacgagaa 2700

ttgttcgcta tgaacagaag ccctgccacc atccctttgg acctgcttag agccagagac 2760

tacggcagtg atgtaaagaa caagagaatt ggtgccatca caaagacaca ggcaacgagt 2820

tggggcgaat acttgacagg aaagatagaa agcttaactg agaggaaagt tgcgacttgt 2880

gtcattcatg gagctggagg ttctggaaaa agtcatgcca tccagaaggc attgagagaa 2940

attggcaagg gctcggacat cactgtagtc ctgccgacca atgaactgcg gctagattgg 3000

agtaagaaag tgcctaacac tgagccctat atgttcaaga cctctgaaaa ggcgttaatt 3060

gggggaacag gcagcatagt catctttgac gattactcaa aacttcctcc cggttacata 3120

gaagccttag tctgtttcta ctctaaaatc aagctaatca ttctaacagg agatagcaga 3180

caaagcgtct accatgaaac tgctgaggac gcctccatca ggcatttggg accagcaaca 3240

gagtacttct caaaatactg ccgatactat ctcaatgcca cacaccgcaa caagaaagat 3300

cttgcgaaca tgcttggtgt ctacagtgag agaacgggag tcaccgaaat cagcatgagc 3360

gccgagttct tagaaggaat cccaactttg gtaccctcgg atgagaagag aaagctgtac 3420

atgggcaccg ggaggaatga cacgttcaca tacgctggat gccaggggct aactaagccg 3480

aaggtacaaa tagtgttgga ccacaacacc caagtgtgta gcgcgaatgt gatgtacacg 3540

gcactttcta gagccaccga taggattcac ttcgtgaaca caagtgcaaa ttcctctgcc 3600

ttctgggaaa agttggacag caccccttac ctcaagactt tcctatcagt ggtgagagaa 3660

caagcactca gggagtacga gccggcagag gcagagccaa ttcaagagcc tgagccccag 3720

acacacatgt gtgtcgagaa tgaggagtcc gtgctagaag agtacaaaga ggaactcttg 3780

gaaaagtttg acagagagat ccactctgaa tcccatggtc attcaaactg tgtccaaact 3840

gaagacacaa ccattcagtt gttttcgcat caacaagcaa aagatgagac tctcctctgg 3900

gcgactatag atgcgcggct caagaccagc aatcaagaaa caaacttccg agaattcctg 3960

agcaagaagg acattgggga cgttctgttt ttaaactacc aaaaagctat gggtttaccc 4020

aaagagcgta ttcctttttc ccaagaggtc tgggaagctt gtgcccacga agtacaaagc 4080

aagtacctca gcaagtcaaa gtgcaacttg atcaatggga ctgtgagaca gagcccagac 4140

ttcgatgaaa ataagattat ggtattcctc aagtcgcagt gggtcacaaa ggtggaaaaa 4200

ctaggtctac ccaagattaa gccaggtcaa accatagcag ccttttacca gcagactgtg 4260

atgctttttg gaactatggc taggtacatg cgatggttca gacaggcttt ccagccaaaa 4320

gaagtcttca taaactgtga gacgacgcca gatgacatgt ctgcatgggc cttgaacaac 4380

tggaatttca gcagacctag cttggctaat gactacacag ctttcgacca gtctcaggat 4440

ggagccatgt tgcaatttga ggtgctcaaa gccaaacacc actgcatacc agaggaaatc 4500

attcaggcat acatagatat taagactaat gcacagattt tcctaggcac gttatcaatt 4560

atgcgcctga ctggtgaagg tcccactttt gatgcaaaca ctgagtgcaa catagcttac 4620

acccatacaa agtttgacat cccagccgga actgctcaag tttatgcagg agacgactcc 4680

gcactggact gtgttccaga agtgaagcat agtttccaca ggcttgagga caaattactc 4740

ctaaagtcaa agcctgtaat cacgcagcaa aagaagggca gttggcctga gttttgtggt 4800

tggctgatca caccaaaagg ggtgatgaaa gacccaatta agctccatgt tagcttaaaa 4860

ttggctgaag ctaagggtga actcaagaaa tgtcaagatt cctatgaaat tgatctgagt 4920

tatgcctatg accacaagga ctctctgcat gacttgttcg atgagaaaca gtgtcaggca 4980

cacacactca cttgcagaac actaatcaag tcagggagag gcactgtctc actttcccgc 5040

ctcagaaact ttctttaacc gttaagttac cttagagatt tgaataagat gtcagcacca 5100

gctagcacaa cacagcccat agggtcaact acctcaacta ccacaaaaac tgcaggcgca 5160

actcctgcca cagcttcagg cctgttcact atcccggatg gggatttctt tagtacagcc 5220

cgtgccatag tagccagcaa tgctgtcgca acaaatgagg acctcagcaa gattgaggct 5280

atttggaagg acatgaaggt gcccacagac actatggcac aggctgcttg ggacttagtc 5340

agacactgtg ctgatgtagg atcatccgct caaacagaaa tgatagatac aggtccctat 5400

tccaacggca tcagcagagc tagactggca gcagcaatta aagaggtgtg cacacttagg 5460

caattttgca tgaagtatgc cccagtggta tggaactgga tgttaactaa caacagtcca 5520

cctgctaact ggcaagcaca aggtttcaag cctgagcaca aattcgctgc attcgacttc 5580

ttcaatggag tcaccaaccc agctgccatc atgcccaaag aggggctcat ccggccaccg 5640

tctgaagctg aaatgaatgc tgcccaaact gctgcctttg tgaagattac aaaggccagg 5700

gcacaatcca acgactttgc cagcctagat gcagctgtca ctcgaggtcg tatcactgga 5760

acaacaaccg ctgaggctgt tgtcactcta ccaccaccat aacagaaact ttctttaacc 5820

gttaagttac cttagagatt tgaataagat ggatattctc atcagtagtt tgaaaagttt 5880

aggttattct aggacttcca aatctttaga ttcaggacct ttggtagtac atgcagtagc 5940

cggagccggt aagtccacag ccctaaggaa gttgatcctc agacacccaa cattcaccgt 6000

gcatacactc ggtgtccctg acaaggtgag tatcagaact agaggcatac agaagccagg 6060

acctattcct gagggcaact tcgcaatcct cgatgagtat actttggaca acaccacaag 6120

gaactcatac caggcacttt ttgctgaccc ttatcaggca ccggagttta gcctagagcc 6180

ccacttctac ttggaaacat catttcgagt tccgaggaaa gtggcagatt tgatagctgg 6240

ctgtggcttc gatttcgaga cgaactcacc ggaagaaggg cacttagaga tcactggcat 6300

attcaaaggg cccctactcg gaaaggtgat agccattgat gaggagtctg agacaacact 6360

gtccaggcat ggtgttgagt ttgttaagcc ctgccaagtg acgggacttg agttcaaagt 6420

agtcactatt gtgtctgccg caccaataga ggaaattggc cagtccacag ctttctacaa 6480

cgctatcacc aggtcaaagg gattgacata tgtccgcgca gggccatagg ctgaccgctc 6540

cggtcaattc tgaaaaagtg tacatagtat taggtctatc atttgcttta gtttcaatta 6600

cctttctgct ttctagaaat agcttacccc acgtcggtga caacattcac agcttgccac 6660

acggaggagc ttacagagac ggcaccaaag caatcttgta caactcccca aatctagggt 6720

cacgagtgag tctacacaac ggaaagaacg cagcatttgc tgccgttttg ctactgactt 6780

tgctgatcta tggaagtaaa tacatatctc aacgcaatca tacttgtgct tgtggtaaca 6840

atcatagcag tcattagcac ttccttagtg aggactgaac cttgtgtcat caagattact 6900

ggggaatcaa tcacagtgtt ggcttgcaaa ctagatgcag aaaccataag ggccattgcc 6960

gatctcaagc cactctccgt tgaacggtta agtttccatt gatactcgaa agaggtcagc 7020

accagctagc aacaaacaag aaatggtgag caagggcgag gagctgttca ccggggtggt 7080

gcccatcctg gtcgagctgg acggcgacgt aaacggccac aagttcagcg tgtccggcga 7140

gggcgagggc gatgccacct acggcaagct gaccctgaag ttcatctgca ccaccggcaa 7200

gctgcccgtg ccctggccca ccctcgtgac caccttcagc tacggcgtgc agtgcttcag 7260

ccgctacccc gaccacatga agcagcacga cttcttcaag tccgccatgc ccgaaggcta 7320

cgtccaggag cgcaccatct tcttcaagga cgacggcaac tacaagaccc gcgccgaggt 7380

gaagttcgag ggcgacaccc tggtgaaccg catcgagctg aagggcatcg acttcaagga 7440

ggacggcaac atcctggggc acaagctgga gtacaactac aacagccaca acgtctatat 7500

catggccgac aagcagaaga acggcatcaa ggtgaacttc aagatccgcc acaacatcga 7560

ggacggcagc gtgcagctcg ccgaccacta ccagcagaac acccccatcg gcgacggccc 7620

cgtgctgctg cccgacaacc actacctgag cacccagtcc gccctgagca aagaccccaa 7680

cgagaagcgc gatcacatgg tcctgctgga gttcgtgacc gccgccggga tcactcacgg 7740

catggacgag ctgtacaagt aatctagcga taccgtcgac tacgtctaca taaccgacgc 7800

ctaccccagt ttcatagtat tttctggttt gattgtatga ataatataaa taaaaaaaaa 7860

aaaaaaaaaa aaaaactagt ggtaccgagc tcttctgtca gcgggcccac tgcatccacc 7920

ccagtacatt aaaaacgtcc gcaatgtgtt attaagttgt ctaagcgtca atttgtttac 7980

accacaatat atcctgccac cagccagcca acagctcccc gaccggcagc tcggcacaaa 8040

atcaccactc gatacaggca 8060

## Description

### FIELD OF THE INVENTION

The present invention relates to a process of high-yield expression of a gene of interest in a plant, in plant tissue or in plant cells using a potexvirus-derived replicon and to a nucleic acid comprising or encoding a potexviral RNA replicon usable for expressing a gene of interest. The invention further relates to a kit of parts comprising two or more vectors that together encode the RNA replicon of the invention.

### BACKGROUND OF THE INVENTION

High-yield expression of heterologous proteins in plants can be achieved using viral vectors. Viral vector systems were predominantly developed for transient expression followed by infection (Donson et al., 1991, Proc Natl Acad Sci U S A, 88:7204-7208; Chapman, Kavanagh & Baulcombe, 1992, Plant J., 2:549-557) or transfection (Marillonnet et al., 2005, Nat Biotechnol., 23:718-723; Santi et al., 2006, Proc Natl Acad Sci U S A. 103:861-866; WO2005/049839) of a plant host. The best-established and commercially viable systems are based on plus-sense single-stranded RNA viruses, preferably on Tobacco Mosaic Virus (TMV)-derived vectors (Kumagai et al., 1994, Proc. Natl. Acad. Sci. USA, 90, 427-430; Mallory et al., 2002, Nature Biotechnol. 20, 622-625; US5316931; US5589367; US5866785; US5977438; WO02088369; WO02097080; WO0229068; US5491076).
Another group of RNA virus-based vectors derived from potexvirus PVX (potato virus X) can also provide for reasonably high yield of recombinant proteins, albeit noticeably lower yield than TMV-derived vectors (Chapman, Kavanagh & Baulcombe, 1992, Plant J., 2:549-557; Baulcombe, Chapman & Santa Cruz, 1995, Plant J., 7:1045-1053; Zhou et al., 2006, Appl. Microbiol. Biotechnol., April 13, epub ahead of print; Zelada et al., 2006, Tuberculosis, 86:263-267). Obviously, such system needs further improvement in order to increase the yield of recombinant protein of interest.
In the first generation of systemic viral vectors, a large proportion of plant resources was wasted for the production of viral coat protein that is necessary for systemic movement of a viral replicon. For TMV-derived vectors this problem was solved by removing the coat protein gene and by using agro-infiltration for efficient systemic delivery of replicons, thus significantly boosting the yield of recombinant proteins of interest (WO2005/049839; Marillonnet et al., (2005), Nat. Biotechnol., 23:718-723). However, unlike TMV-derived replicons, potexvirus-derived replicons require viral coat protein not only for systemic, but also for short distance (cell-to-cell) movement. Therefore, the coat protein gene of potexvirus-derived viral vectors cannot be removed without a severe loss of protein expression efficiency. Further, engineering of plant host providing viral coat protein *in trans* is rarely a good solution because of gene silencing. Also, transgenic plants expressing coat protein might exhibit coat protein-mediated resistance to challenges by plant viruses (Beachy, RN., 1999, Philos. Trans. R. Soc. Lond B Biol. Sci., 354:659-664; Wisniewski et al., 1990, Plant Cell, 2:559-567). Another similar phenomenon called heterologous CP-mediated resistance can be a problem, for example when a transgenic plant expressing PVX CP reduces cell-to-cell spread of TMV RNA (Bazzini et al., 2006, J. Gen. Virol., 87:1005-1012). In addition, expression of PVX coat protein in transgenic tobacco plants rescues movement-deficient PVX, but compromises the efficiency of cell-to-cell movement and viral replication (Spillane et al., 1997, Virology, 236:76-84). This could be an issue when the use of two viral vectors (e.g. TMV- and PVX-based vectors) in the same plant cell is required for expression of hetero-oligomeric proteins (e.g. for the co-expression of the heavy and light chains of a monoclonal antibody) (WO2006/079546; Giritch et al., 2006, Proc. Natl. Acad. Sci. USA, in press).

### GENERAL DESCRIPTION OF THE INVENTION

Therefore, it is an object of the invention to provide a potexvirus-based viral vector for high yield expression of a protein of interest in plants, in plant tissue or in plant cells. The viral vector should be capable of cell-to-cell movement and should waste as little resources of a plant host for producing viral coat protein as possible.
This object is solved by a nucleic acid comprising or encoding an RNA replicon comprising, in this order, the following segments:
(i) a nucleic acid sequence encoding an RNA-dependent RNA polymerase or a function-conservative derivative thereof;
(ii) a nucleic acid sequence encoding one or more proteins required for cell-to-cell movement of said replicon in a plant or in plant tissue;
(iii) a heterologous nucleic acid sequence expressible from said replicon in a plant or in plant tissue;
or a complementary sequence thereof.

Said RNA replicon is preferably a replicon for replicating and expressing said heterologous nucleic acid sequence in a plant or in plant tissue. Said RNA replicon is preferably built on a potexvirus and uses the replication and expression system of a potexvirus for expressing a heterologous nucleic acid sequence.
The invention further provides a kit of parts comprising at least two vectors (pro-vectors) that are capable of assembling said nucleic acid of the invention in plant cells by site-directed recombination.
The invention also provides a process of expressing a heterologous nucleic acid sequence of interest in a plant or in plant tissue, comprising providing a plant or plant tissue with said nucleic acid of the invention. The invention also provides a process of expressing a heterologous nucleic acid sequence of interest in a plant or in plant tissue, comprising providing a plant or plant tissue with two or more vectors that are capable of assembling said nucleic acid of the invention in plant cells by site-directed recombination.

The inventors have surprisingly identified a way to increase the expression yield of a protein of interest expressed in a plant or in plant tissue from a potexviral vector by a vector design wherein the sequences as defined in item (ii) are positioned after (downstream in 5 to 3' direction) the RNA-dependent RNA polymerase coding sequence (RdRp) of item (i) and precede said heterologous nucleic acid sequence of item (iii). In the special case of potexviral vectors, this vector design leads to a cell-to-cell movement capability of the RNA replicon and, at the same time, to higher expression levels of the heterologous nucleic acid compared to conventional potexviral vectors where a heterologous nucleic acid was placed upstream of the potexviral coat protein gene. The effect identified by the inventors may be due to the fact that the position of the sequences of item (ii) leads to lower expression levels of these proteins and to less consumption of plant resources for the expression of these proteins, allowing higher expression levels of the 3' protein of interest. Importantly, the limited expression level of potexviral coat protein in the invention is sufficient for supporting efficient cell-to-cell movement for high-yield expression of the heterologous sequence of interest.

Potexviruses are plant RNA viruses with a plus-sense single-stranded genome. Thus, said nucleic acid of the invention may be RNA being or comprising said RNA replicon or may be DNA encoding said RNA replicon. Herein, the terms "potexviral vector" or "potexviral replicon" mean that the vector or replicon make use of the replication and protein expression system of potexviruses. Said nucleic acid may be built on a natural potexvirus e.g. by using genetic components from a potexvirus. Said nucleic acid of the invention may be obtainable by inserting said heterologous nucleic acid sequence into a nucleic acid construct encoding a potexvirus, whereby said heterologous nucleic acid sequence of interest is inserted downstream of a sequence enocoding the coat protein of said potexvirus. However, various modifications may be made to the various genetic components of a natural potexvirus, such as to the RdRP gene, the triple gene block, the coat protein gene, or to the 5' or 3' non-translated regions of a potexvirus, examples for which are described below.

Said RNA replicon of the invention comprises, in the order from the 5' end to the 3' end, said segments (i) to (iii) of the invention. Further genetic elements will typically be present on said replicon for replication and expression. For being an RNA replicon, i.e. for autonomous replication in a plant cell, said RNA replicon encodes an RdRp or a function-conservative derivative thereof. Said RNA replicon preferably further has potexviral 5'- or 3'-untranslated regions and promoter-sequences in the 5'- or 3'-untranslated regions of said RNA replicon for binding said RdRp and for replicating said RNA replicon. Said RNA replicon further may have sub-genomic promoters in segments of item (ii) and (iii) for generating sub-genomic RNAs for the expression of the proteins encoded by the segments of items (ii) and (iii). If said nucleic acid is DNA, it will typically have a transcription promoter for allowing production by transcription of said RNA replicon in vitro or in planta. An example of a transcription promoter allwowing transcription of said RNA replicon from a DNA nucleic acid in planta is the 35S promoter that is widely used in plant biotechnology.

Said segment (i) may encode an RdRp of a potexvirus such as potato virus X, or a function-conservative variant thereof. The RdRp used in said RNA replicon may be considered a function-conservative variant of a potexviral RdRp if said sequence of item (i) encodes a protein having a sequence identity of at least 36 % to a protein encoded by SEQ ID NO:4. In another embodiment, said sequence identity is at least 45 %, preferably at least 55%, more preferably at least 65% and most preferably at least 75% to a protein encoded by SEQ ID NO:4. These sequence identities may be present over the entire sequence of SEQ ID NO:4. Alternatively, these sequence identities may be present within a protein sequence segment of at least 300 amino acid residues, preferably at least 500 amino acid residues, more preferably at least 900 amino acid residues, most preferably 1400 amino acid residues. Amino acid sequence identities may be determined using BLASTX 2.2.14 using the standard settings. The standard settings allow, for example, for sequence gaps in alignments.

Alternatively, the RdRp used in said RNA replicon may be considered a function-conservative variant of a potexviral RdRp if said sequence of item (i) encodes a protein having a sequence homology of at least 50 % to a protein encoded by SEQ ID NO:4. In another embodiment, said sequence homology is at least 60 %, preferably at least 70%, and more preferably at least 80 % to a protein encoded by SEQ ID NO:4. These sequence homologies may be present over the entire sequence of SEQ ID NO:4. Alternatively, these sequence homologies may be present within a protein sequence segment of at least 300 amino acid residues, preferably at least 500 amino acid residues, more preferably at least 900 amino acid residues, most preferably at least 1400 amino acid residues. Amino acid sequence homologies may be determined using BLASTX 2.2.14 using the standard settings. The standard settings allow, for example, for sequence gaps in alignments.

Alternatively, the RdRp used in said RNA replicon may be considered a function-conservative variant of a potexviral RdRp if said sequence of item (i) has a sequence identity of at least 55 %, preferably at least 60%, more preferably at least 70% to SEQ ID NO: 4. Said sequence identities may be present within SEQ ID NO:4 or within a sequence segment of at least 900 nucleotides, preferably at least 1500 nucleotides, more preferably of at least 2000 nucleotides and most preferably of at least 4200 nucleotides. Nucleotide sequence identities may be determined using BLASTN 2.2.14 using the standard settings. The standard settings allow, for example, for sequence gaps in the alignments.

Said RNA replicon comprises said nucleic acid sequence of item (ii) for allowing cell-to-cell movement of said RNA replicon in a plant or in plant tissue. Cell-to-cell movement of said RNA replicon is important for achieving expression of the sequence of item (iii) in as many cells of said plant or said tissue as possible. Said nucleic acid sequence of item (ii) may comprise the potexviral triple gene block TGB or a function-conservative variant thereof (a review on the TGB is found in J. Gen. Virol. (2003) 84, 1351-1366). The potexviral triple gene block encodes for three proteins necessary to provide the capability of cell-to-cell movement to a potexvirus. Thus, a variant of said TGB is considered to be a function-conservative variant of TGB if the variant can provide, optionally with other necessary components, the RNA replicon of the invention with the capability of cell-to-cell movement in a plant or in plant tissue.

Said nucleic acid sequence of item (ii) may comprise a further sequence encoding a protein necessary for cell-to-cell movement of said RNA replicon such as a potexviral coat protein or a function-conservative variant thereof. A variant of said potexviral coat protein is considered a function-conservative variant of said coat protein if it is capable of providing said RNA replicon, together with other necessary components such as the TGB, with the capability of cell-to-cell movement in a plant or in plant tissue. In one embodiment where said RNA replicon comprises a potexviral coat protein (or a function-conservative variant thereof), said RNA replicon does not have an origin of viral particle assembly for avoiding spread of said RNA replicon from plant to plant in the form of an assembled plant virus. If said RNA replicon comprises a potexviral coat protein (or a function-conservative variant thereof) and a TGB (or a function-conservative variant thereof), it is possible that said TGB is located upstream of said coat protein gene or vice versa.
Alternatively, said nucleic acid sequence of item (ii) may comprise, optionally instead of said potexviral coat protein, a sequence encoding a plant viral movement protein (MP). An example of a suitable MP is a tobamoviral MP such as an MP of tobacco mosaic virus.

Said heterologous nucleic acid sequence of item (iii) typically comprises the coding sequence of a protein of interest to be expressed in a plant or in plant tissue. Such coding sequence of item (iii) is also referred to herein as gene of interest. Said sequence of item (iii) is heterologous to said plant virus on which said RNA replicon is based. In many cases, said sequence is also heterologous to said plant or said plant tissue in which it is to be expressed. For being expressible from said RNA replicon in a plant or in plant tissue, said sequence of item (iii) typically comprises a sub-genomic promoter and other sequences required for expression such as ribosome binding site and/or an internal ribosome entry site (IRES). In a preferred embodiment, said heterologous nucleic acid sequence of item (iii) has one gene of interest that codes for one protein of interest.

The process of the invention can be used for producing one protein of interest or more than one protein of interest in a plant or in plant tissue. Said process comprises providing a plant, plant tissue or plant cells with said nucleic acid of the invention. The process of the invention is preferably performed in plants or in plant tissue. In one embodiment, said process is a transient expression process, whereby incorporation of the nucleic acid of the invention into chromosomal DNA of the plant host is not necessary.

If said nucleic acid of the invention is RNA, it may be used for infecting a plant or plant tissue, preferably in combination with mechanical injury of infected plant tissue such as leaves. In another embodiment, said nucleic acid of the invention is DNA and said DNA may be introduced into cells of a plant or plant tissue, e.g. by particle bombardment or by Agrobacterium-mediated transformation. Agrobacterium-mediated transformation is the method of choice if several plants are to be provided with said nucleic acid of the invention, i.e. for large scale protein production methods.
The process of the invention may be performed using the pro-vector approach (WO02088369; Marillonnet et al., 2004, Proc. Natl. Acad. Sci. USA, 101:6852-6857) by providing a plant or plant tissue with said kit or parts of the invention. In cells of said plant, the nucleic acid of the invention is then produced by site-specific recombination between pro-vectors. In one embodiment, a first vector (pro-vector) comprising or encoding segments of items (i) and (ii) and a second vector (pro-vector) comprising or encoding the segment of item (iii) is provided to a plant or plant tissue, wherein said first and said second pro-vector each has a recombination site for allowing assembly of a nucleic acid of the invention by site-specific recombination between said first and said second pro-vector. Two or more vectors may be provided to a plant or to plant tissue by providing mixtures of the vectors or mixtures of Agrobacterium strains, each strain containing one of said vectors, to a plant or to plant tissue.

Said one or more than one protein of interest may be purified after production in said plant or plant tissue. Methods or purifying proteins from plants or plant cells are known in the art. In one method, a protein of interest may be directed to a plant apoplast and purified therefrom as described in WO 03/020938.
If one protein of interest has to be produced, a nucleotide sequence coding for said protein of interest may be included in said nucleotide sequence encoding said RNA replicon. If two or more proteins of interest are to be produced in the same plant or in the same plant tissue, said plant or plant cells may be provided with a second nucleic acid comprising or encoding a second or further RNA replicon. Said further RNA replicon may then encode one or more further proteins of interest. In one embodiment, a first and a second nucleic acid of the invention may comprise or encode non-competing RNA replicons as described in WO2006/079546.

The present invention may in principle be applied to any plants for which infectious potexviruses exist and for which viral vector systems were established. Preferred plants are *Nicotiana* species like *Nicotiana benthamiana* and *Nicotiana tabacum;* preferred plant species other than *Nicotiana* species are *Petunia hybrida, Brassica campestris, B. juncea,* cress, arugula, mustard, Strawberry spinach, *Chenopodium capitatum,* alfalfa, lettuce, sunflower, potato and cucumber. The most preferred plant RNA viruses the RNA replicons of the invention may be based on are Potexviruses such as potato virus X (PVX), papaya mosaic potexvirus or bamboo mosaic potexvirus.
The major application of the present invention is the production of a protein of interest in plants, plant leaves or plant tissue or cell culture. If the process of the invention is performed in plants, plants that do not enter the human or animal food chain are preferred, like *Nicotiana* species. Plants that do not enter the human or animal food chain can be cultivated in an open field and harvested within certain period after infection with said RNA replicon. Preferably, whole plants or plant parts shall be confined to a contained environment, e.g. a glasshouse or a designed chamber for the incubation period necessary to provide for desired level of expression.
The efficiency of the production process of the present invention is such that a new dimension in plant expression systems is attained. The expression levels achievable with the present invention are such that expenditures for downstream processing (including separation and purification of the protein of interest) are low enough to make the process of the invention competitive with other large-scale expression systems. The invention provides the first high-yield potexviral plant expression system that can be used on a large scale.

### BRIEF DESCRIPTION OF THE FIGURES

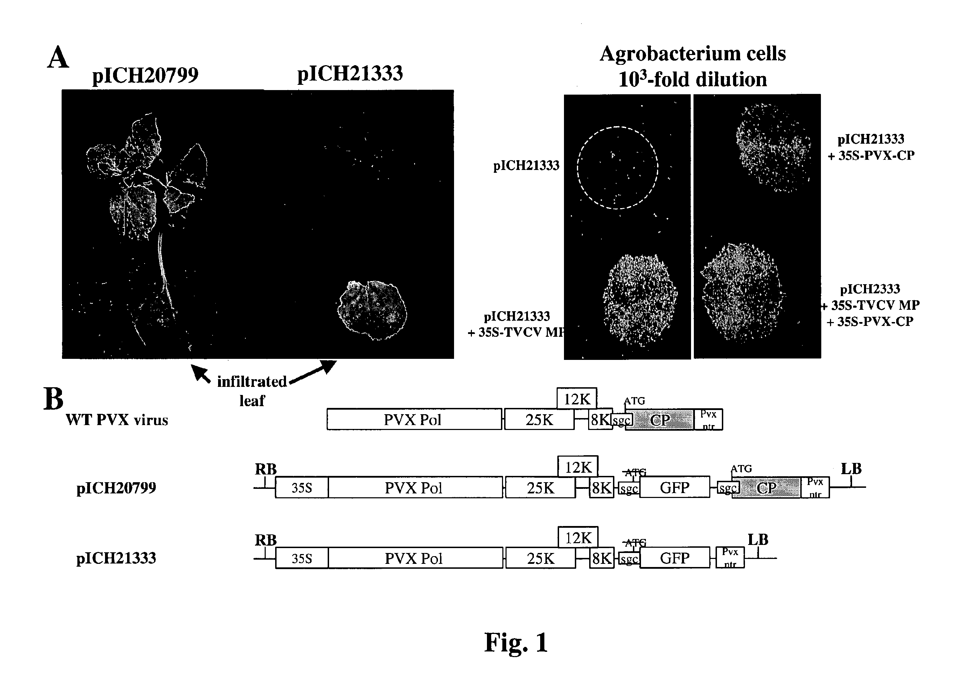

Figure 1. A - *N. benthamiana* plants (left picture) and plant leaves (right picture) monitored under UV light after agro-infiltration with different PVX vectors. 35S-PVX-CP: vector providing PVX coat protein (CP) under control of CaMV 35S promoter; 35S-TVCV MP: vector providing tobamovirus Turnip Vein Clearing Virus movement protein (MP) under control of CaMV 35S promoter. B - schematic presentation of T-DNA regions of constructs plCH20799 and plCH21333. PVX Pol: potato virus X RNA-dependent RNA polymerase; 35S: CaMV 35S promoter; 25K, 12K, 8K: triple gene block; CP: PVX coat protein; PVX ntr: PVX non-translated region; GFP: jellyfish green fluorescent protein; sgc: subgenomic promoter of coat protein gene.

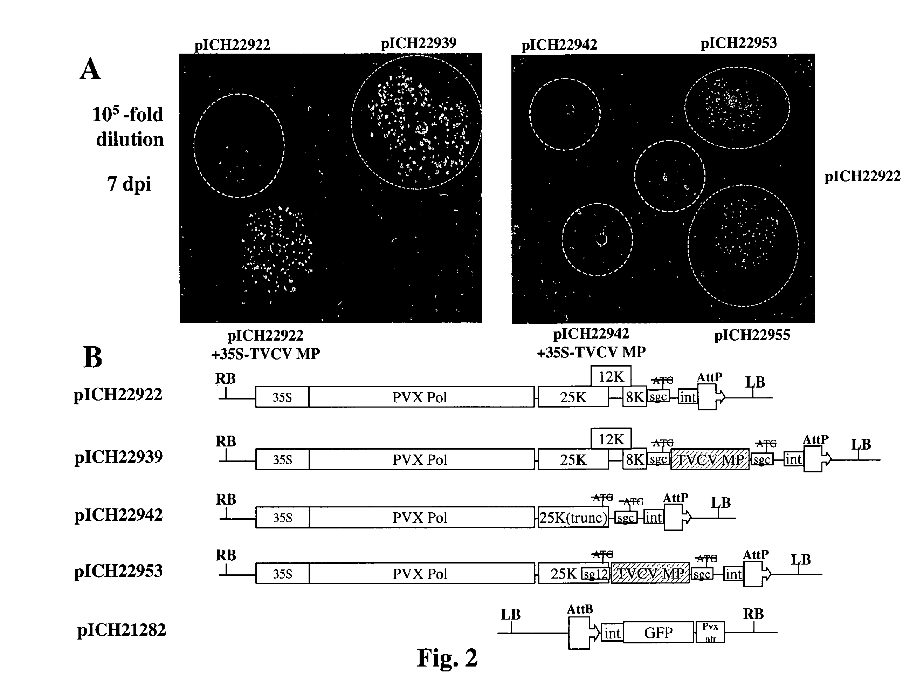

Figure 2. Expression of GFP using different PVX provector modules. A - *N. benthamiana* leaves monitored under UV light after agro-infiltration with different 5' PVX provectors in combination with 3' provector plCH21282 and in the presence of phage C31 integrase providing for site-specific recombination-mediated assembly of viral replicon-encoding DNA. Only 5' provectors (e.g. plCH22922; plCH22942, etc.) mark the inoculation spots. 35S-TVCV MP: vector providing tobamovirus Turnip Vein Clearing Virus movement protein (MP) under control of the CaMV 35S promoter. The pictures were taken 7 days post infiltration (7 dpi). Agrobacterium suspensions used for infiltration were diluted 10⁵ fold for achieving an expression level allowing visual detection of differences in expression levels.
B depicts T-DNA regions of plasmids plC22922, plCH22939, plCH22942, plCH22953 and plCH21282. PVX Pol: potato virus X RNA-dependent RNA polymerase; 35S: CaMV 35S promoter; 25K, 12K, 8K: triple gene block; 25K (trunk): truncated gene encoding for 25K protein; CP: PVX coat protein; Pvx ntr: PVX non-translated region; GFP: jellyfish green fluorescent protein; sgc: subgenomic promoter of coat protein gene; int: 5' end of plant intron; AttP and AttB: sequences recognized by site-specific integrase of phage C31.

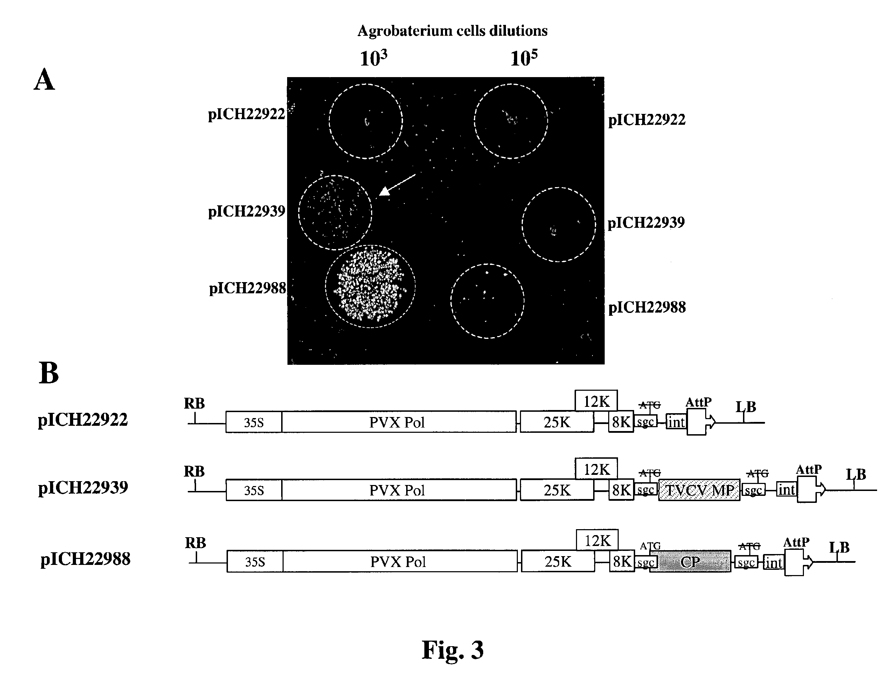

Figure 3. Comparison of GFP expression using PVX provector modules that do not (plCH22922) or do provide (plCH22939 or plCH22988) for cell-to-cell movement in plant tissue. A - *N. benthamiana* leaf monitored under UV light after agro-infiltration with different 5' PVX provectors in combination with 3' provector plCH21282 and in the presence of phage C31 integrase providing for site-specific recombination mediated assembly of viral replicon-encoding DNA. The picture was taken 7 days post infiltration (7 dpi). Agrobacterium suspensions used for infiltration were diluted 10³ or 10⁵ fold as indicated.
B - depicts T-DNA regions of plasmids plC22922, plCH22939 and plCH22988 plCH22953 and plCH21282. PVX Pol: potato virus X RNA-dependent RNA polymerase; 35S: CaMV 35S promoter; 25K, 12K, 8K: triple gene block; 25K (trunk): truncated gene encoding for 25K protein; TVCV MP: tobamovirus Turnip Vein Clearing Virus movement protein; CP: PVX coat protein; Pvx ntr: PVX non-translated region; GFP: jellyfish green fluorescent protein; sgc: subgenomic promoter of coat protein gene; int: 5' end of plant intron; AttP and AttB: sequences recognized by site-specific integrase of phage C31.

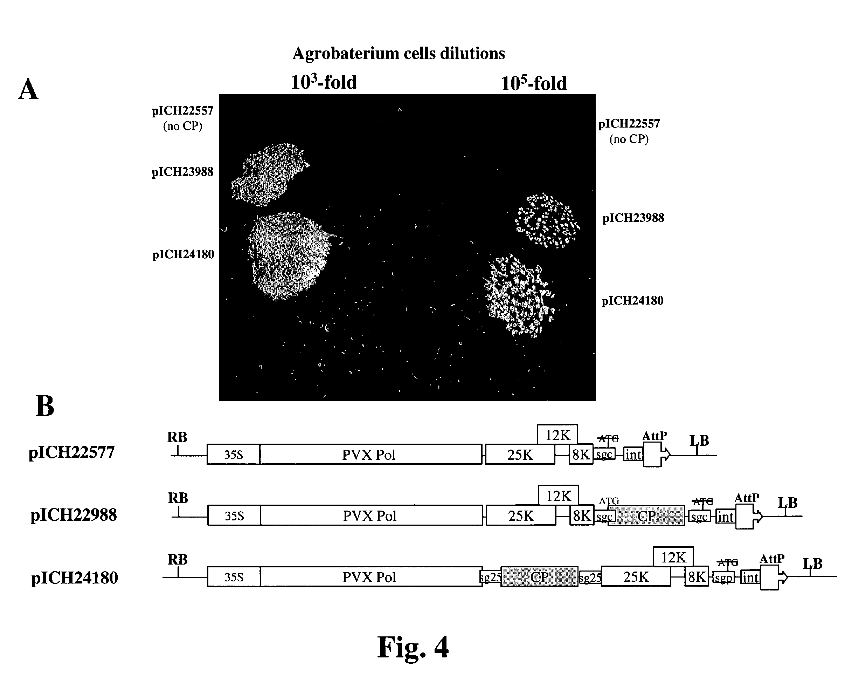

Figure 4. Comparison of GFP expression using PVX provector modules that do not (plCH22577) or do provide (plCH22988 or plCH24180) for cell-to-cell movement. A - *N*. *benthamiana* leaf monitored under UV light after agro-infiltration with different 5' PVX provectors in combination with 3' provector plCH21282 and in the presence of phage C31 integrase providing for site-specific recombination-mediated assembly of viral replicon-encoding DNA. The picture was taken 7 days post infiltration (7 dpi). Agrobacterium suspensions used for infiltration were diluted 10³ and 10⁵ fold as indicated.
B - depicts T-DNA regions of plasmids plC22577, plCH22988 and plCH24180. PVX Pol: potato virus X RNA-dependent RNA polymerase; 35S: CaMV 35Spromoter; 25K, 12K, 8K: triple gene block; CP: PVX coat protein; PVX ntr: PVX non-translated region; sgc: subgenomic promoter of coat protein gene; sg25: subgenomic promoter of 25K gene; int: 5' end of plant intron; AttP: sequence recognized by site-specific integrase of phage C31.

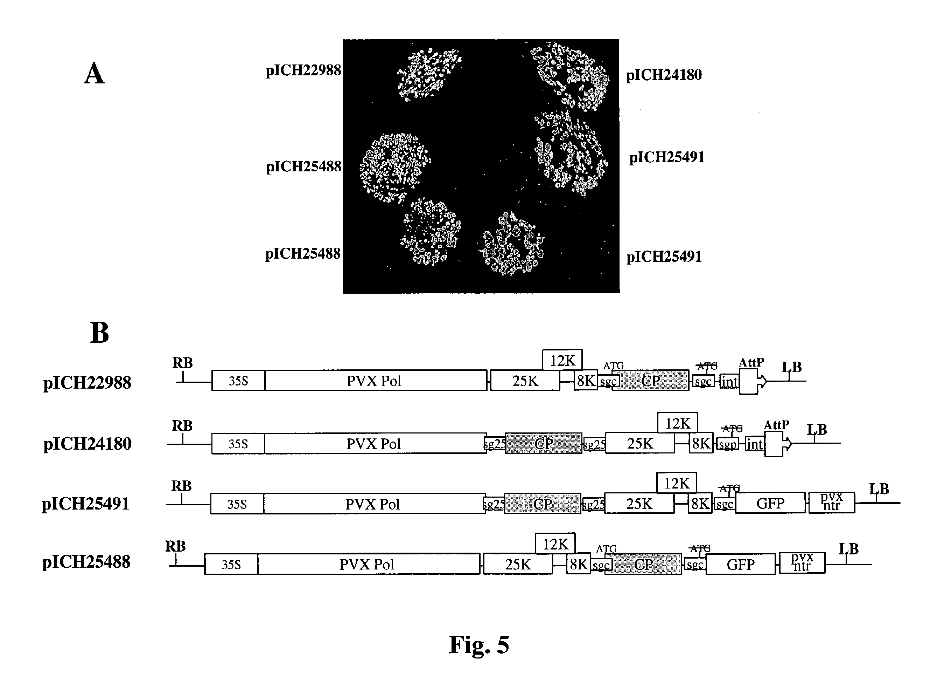

Figure 5. GFP expression using different PVX expression cassettes (provector modules and assembled vectors) with CP located at different positions in front of the GFP gene. A *- N. benthamiana* leaf monitored under UV light after agro-infiltration with assembled viral vectors plCH25491, plCH25488 or different 5' PVX provectors (plCH22988 or plCH24180) in combination with 3' provector plCH21282 and in the presence of phage C31 integrase providing for site-specific recombination-mediated assembly of viral replicon-encoding DNA. The picture was taken 7 days post infiltration. Agrobacterium suspension used for infiltration were diluted 10⁵ fold
B - depicts T-DNA regions of plasmids plC22988, plCH25491, plCH25488 and plCH24180. PVX Pol: potato virus X RNA-dependent RNA polymerase; 35S: CaMV 35Spromoter; 25K, 12K, 8K: triple gene block; CP: PVX coat protein; Pvx ntr: PVX non-translated region; sgc: subgenomic promoter of coat protein gene; sg25: subgenomic promoter of 25K gene; int: 5' end of plant intron; AttP: sequence recognized by site-specific integrase of phage C31.

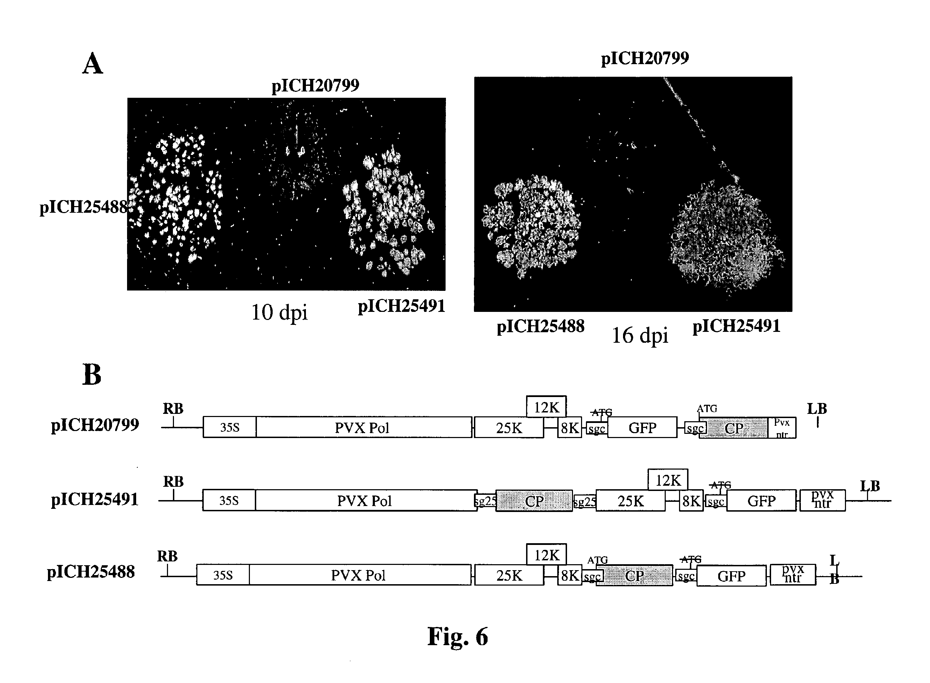

Figure 6. GFP expression using assembled PVX expression cassettes with CP located at different positions in front of the GFP gene. A - *N. benthamiana* leaves monitored under UV light after agro-infiltration with assembled viral vectors plCH25491, plCH25488 and plCH20799 ten (left panel) and sixteen (right panel) days post-infiltration. Agrobacterium suspensions used for infiltration were diluted 10⁵ fold.
B - depicts T-DNA regions of plasmids plC20799, plCH25491, and plCH25488. PVX Pol: potato virus X RNA-dependent RNA polymerase; 35S: CaMV 35Spromoter; 25K, 12K, 8K: triple gene block; CP: PVX coat protein; PVX ntr: PVX non-translated region; sgc: subgenomic promoter of coat protein gene; sg25: subgenomic promoter of 25K gene.

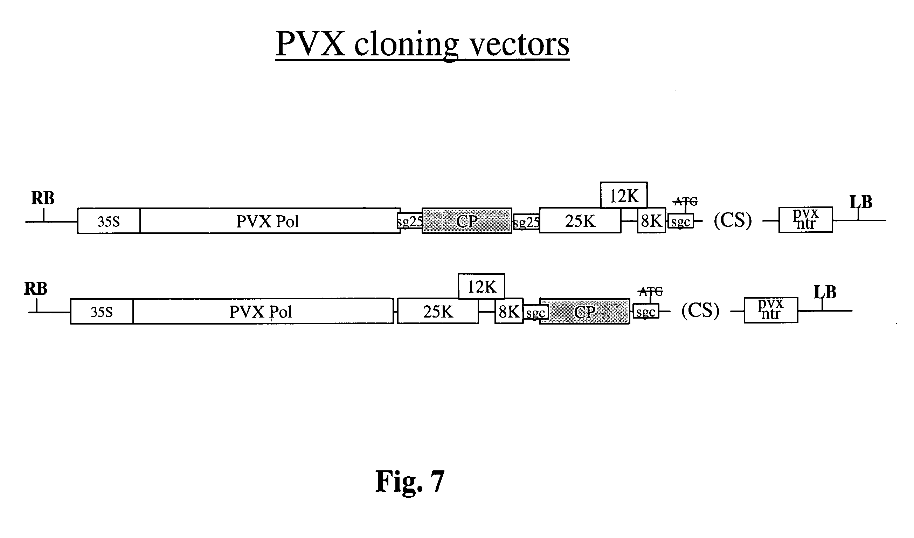

Figure 7 depicts PVX cloning vectors with multiple cloning sites (CS). PVX Pol: potato virus X RNA-dependent RNA polymerase; 35S: CaMV 35Spromoter; 25K, 12K, 8K: triple gene block; CP: PVX coat protein; PVX ntr: PVX non-translated region; sgc: subgenomic promoter of coat protein gene; sg25: subgenomic promoter of 25K gene.

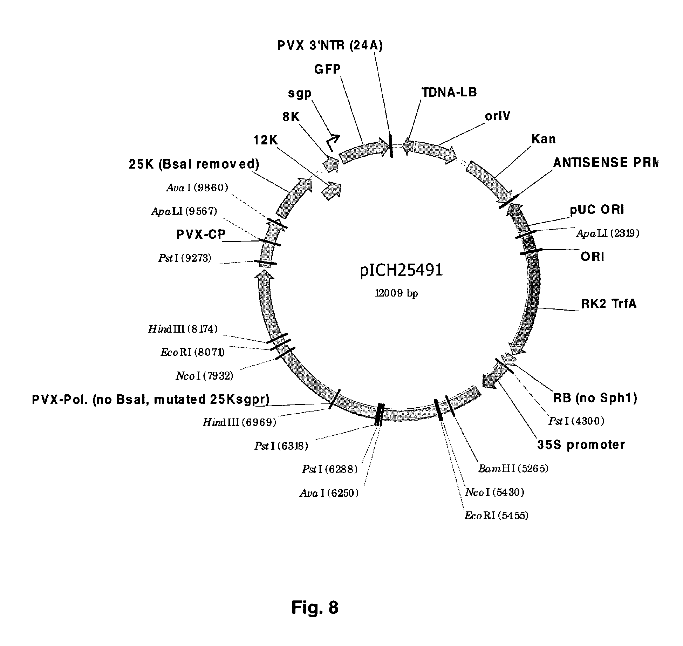

Figure 8 shows a map of plCH25491. The nucleotide sequence of the T-DNA region of plCH25491 is given in SEQ ID NO:6.

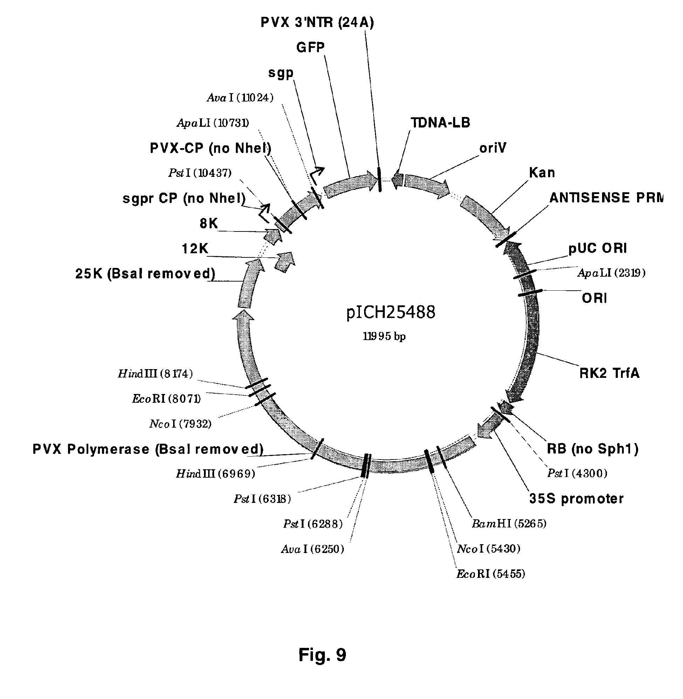

Figure 9 shows a map of plCH25488. The nucleotide sequence of the T-DNA region of plCH25488 is given in SEQ ID NO:5.

### DETAILED DESCRIPTION OF THE INVENTION

This invention describes a new design for potexvirus-derived RNA replicons that improves yield of a protein of interest to be expressed from said RNA replicons in a plant or in plant tissue. The process of the invention has better biosafety features than conventional potexvirus-derived RNA replicons, as it produces less viral coat protein and consequently, can form less viral particles.
We have surprisingly found that the position of a coding sequence for a coat protein or heterologous movement protein that precedes said heterologous nucleic acid sequence (iii) encoding a protein of interest increases the yield of said recombinant protein at the expense of viral coat protein. The PVX coat protein is necessary not only for systemic, but also together with three other proteins of triple gene block for cell-to-cell (short distance) movement (Yang et al., 2000, Mol. Plant Microbe Interact., 13:599-605; Fedorkin et al., 2001, J. Gen. Virol., 82:449-458). Herein, it is shown (see example 1 and Fig. 1) that the absence of PVX coat protein makes PVX-derived RNA replicons deficient in long distance (Fig. 1 A, left panel) and cell-to-cell (Fig. 1 A, right panel) movement. Cell-to cell movement can be restored by providing *in trans* PVX coat protein (CP) or tobamoviral movement protein tobacco mosaic virus (TMV) movement protein (MP) (Fig. 1A, right panel). It is also shown (example 2, Fig. 2) that a PVX-derived RNA replicon with a TVCV movement protein is capable of short distance (cell-to-cell) movement. The experiment was carried out using the pro-vector system (Marillonnet et al., 2004, Proc. Natl. Acad. Sci. USA, 101:6852-6857) that allows assembly *in planta* of DNA precursor of RNA replicon via site-specific recombination e.g. between AttP and AttB sites of provectors (Fig. 2B).
A comparative analysis of PVX-derived RNA replicons with either TVCV MP (plCH22939) or PVX CP is described in example 3 and shown in Fig. 3. As in the case described above, the provector system was used. It is evident from Fig. 3 that PVX CP positioned in front of the gene of interest provides for cell-to-cell movement of said RNA replicon. As it follows from the brightness and density of GFP spots (Fig. 3A), the efficiency of cell-to-cell movement is significantly higher than with TVCV MP.
In another embodiment of this invention, the effect of the position of PVX CP preceding the gene of interest was tested. It is evident from Fig. 4A that the location of CP after the triple gene block provides for a more efficient cell-to-cell movement than location of said CP between PVX RNA dependent RNA polymerase and the triple gene block. The provector system was also used in this experiment. No difference in the expression level of the protein of interest (GFP) was found between the cases where provectors or assembled vectors providing for PVX-derived RNA replicons were used (see Example 5, Fig. 5). When we included in comparative studies vectors that provide for RNA replicon containing CP after the gene of interest, the expression level (brightness of GFP expressing area) of said gene of interest from such vector was surprisingly significantly weaker compared with vectors where CP was positioned before the gene of interest (plCH20799 versus plCH25488 or plCH25491, Fig. 6, example 6).

In the examples, we use PVX-based RNA replicons. However, other viruses belonging to potexvirus taxonomic group can be used for the construction of RNA virus-based vectors according to the present invention. Viral species usable for the invention include but not limited to bamboo mosaic potexvirus, papaya mosaic potexvirus, alternanthera mosaic potexvirus, clover yellow mosaic virus, plantain virus X, white clover mosaic virus and potato aucuba mosaic virus. For details on plant viral movement proteins, see the recent review of WJ Lucas (2006, Virology, 344:169-184).

One RNA replicon derived from one plant virus may be used in the process of the invention, e.g. from potato virus X (PVX). However, two or more different RNA replicons may be used in a plant or plant tissue for expressing two different proteins of interest, whereby such different RNA replicons are preferably derived from different plant viruses. Such different plant viruses from which said different RNA replicons may be derived are preferably synergistic or non-competing viruses. "Synergistic" and "non-competing" are used herein synonymously. Synergistic viruses can coexist and efficiently amplify in the same plant cells. Similarly, RNA replicons derived from synergistic RNA viruses can coexist and efficiently amplify in the same plant cells. An example of such a synergistic pair of RNA replicons is a pair of RNA replicons, whereby one RNA replicon is derived from TMV or TVCV and the other RNA replicon is derived from a potexvirus such as PVX. Synergistic RNA replicons may be used for the expression of two or more proteins or protein subunits of interest, such as the heavy and the light chain of a monoclonal antibody, in the same plant cell. Processes of expressing two or more proteins of interest in the same plant or in the same plant cells using different (non-competing) viral vectors is described in EP1686176.

In the examples, we predominantly used *Agrobacterium-mediated* delivery of viral vectors in plant cells. Various other methods usually used for stable transformation of plants may be used for the delivery of vectors into plant cells such as direct introduction of a heterologous nucleotide sequence into cells by means of microprojectile bombardment, electroporation or PEG-mediated transformation of protoplasts. *Agrobacterium-mediated* plant transformation is preferred. Thus, a heterologous nucleotide sequence may be transformed into plant cells by various technologies such as by a Ti-plasmid vector carried by *Agrobacterium* (US 5,591,616; US 4,940,838; US 5,464,763), particle or microprojectile bombardment (US 05100792; EP 00444882B1; EP 00434616B1). In principle, other plant transformation methods can also be used e.g. microinjection (WO 09209696; WO 09400583A1; EP 175966B1), electroporation (EP00564595B1; EP00290395B1; WO 08706614A1), etc. The choice of the transformation method depends inter alia on the plant species to be transformed. For example, microprojectile bombardment may be preferred for monocot transformation, while for dicots, Agrobacterium-mediated transformation gives generally better results.

The present invention is preferably carried out with higher, multi-cellular plants. Preferred plants for the use in this invention include any plant species with preference given to agronomically and horticulturally important species. Common crop plants for the use in present invention include alfalfa, barley, beans, canola, cowpeas, cotton, corn, clover, lotus, lentils, lupine, millet, oats, peas, peanuts, rice, rye, sweet clover, sunflower, sweetpea, soybean, sorghum triticale, yam beans, velvet beans, vetch, wheat, wisteria, and nut plants. The plant species preferred for practicing of this invention are including but not restricted to:
Representatives of Graminae, Compositae, Solanacea and Rosaceae. Additionally, preferred species for use in the invention, as well as those specified above, plants from the genera: Arabidopsis, Agrostis, Allium, Antirrhinum, Apium, Arachis, Asparagus, Atropa, Avena, Bambusa, Brassica, Bromus, Browaalia, Camellia, Cannabis, Capsicum, Cicer, Chenopodium, Chichorium, Citrus, Coffea, Coix, Cucumis, Curcubita, Cynodon, Dactylis, Datura, Daucus, Digitalis, Dioscorea, Elaeis, Eleusine, Festuca, Fragaria, Geranium, Glycine, Helianthus, Heterocallis, Hevea, Hordeum, Hyoscyamus, Ipomoea, Lactuca, Lens, Lilium, Linum, Lolium, Lotus, Lycopersicon, Majorana, Malus, Mangifera, Manihot, Medicago, Nemesia, Nicotiana, Onobrychis, Oryza, Panicum, Pelargonium, Pennisetum, Petunia, Pisum, Phaseolus, Phleum, Poa, Prunus, Ranunculus, Raphanus, Ribes, Ricinus, Rubus, Saccharum, Salpiglossis, Secale, Senecio, Setaria, Sinapis, Solanum, Sorghum, Stenotaphrum, Theobroma, Trifolium, Trigonella, Triticum, Vicia, Vigna, Vitis, Zea, and the Olyreae, the Pharoideae and many others.

In one specific embodiment of the process of the invention, RNA replicons derived from PVX are used with Nicotiana plants.

Proteins of interest, or fragments thereof, that can be expressed, in sense or antisense orientation, using the invention include: starch modifying enzymes (starch synthase, starch phosphorylation enzyme, debranching enzyme, starch branching enzyme, starch branching enzyme II, granule bound starch synthase), sucrose phosphate synthase, sucrose phosphorylase, polygalacturonase, polyfructan sucrase, ADP glucose pyrophosphorylase, cyclodextrin glycosyltransferase, fructosyl transferase, glycogen synthase, pectin esterase, aprotinin, avidin, bacterial levansucrase, *E.coli* glgA protein, MAPK4 and orthologues, nitrogen assimilation/methabolism enzyme, glutamine synthase, plant osmotin, 2S albumin, thaumatin, site-specific recombinase/integrase (FLP, Cre, R recombinase, Int, SSVI Integrase R, Integrase phiC31, or an active fragment or variant thereof), isopentenyl transferase, Sca M5 (soybean calmodulin), coleopteran type toxin or an insecticidally active fragment, ubiquitin conjugating enzyme (E2) fusion proteins, enzymes that metabolise lipids, amino acids, sugars, nucleic acids and polysaccharides, superoxide dismutase, inactive proenzyme form of a protease, plant protein toxins, traits altering fiber in fiber producing plants, Coleopteran active toxin from *Bacillus thuringiensis* (Bt2 toxin, insecticidal crystal protein (ICP), CrylC toxin, delta endotoxin, polyopeptide toxin, protoxin etc.), insect specific toxin AalT, cellulose degrading enzymes, E1 cellulase from *Acidothermus celluloticus,* lignin modifying enzymes, cinnamoyl alcohol dehydrogenase, trehalose-6-phosphate synthase, enzymes of cytokinin metabolic pathway, HMG-CoA reductase, *E*. *coli* inorganic pyrophosphatase, seed storage protein, *Erwinia herbicola* lycopen synthase, ACC oxidase, pTOM36 encoded protein, phytase, ketohydrolase, acetoacetyl CoA reductase, PHB (polyhydroxybutanoate) synthase, acyl carrier protein, napin, EA9, non-higher plant phytoene synthase, pTOM5 encoded protein, ETR (ethylene receptor), plastidic pyruvate phosphate dikinase, nematode-inducible transmembrane pore protein, trait enhancing photosynthetic or plastid function of the plant cell, stilbene synthase, an enzyme capable of hydroxylating phenols, catechol dioxygenase, catechol 2,3-dioxygenase, chloromuconate cycloisomerase, anthranilate synthase, *Brassica* AGL15 protein, fructose 1,6-biphosphatase (FBPase), AMV RNA3, PVY replicase, PLRV replicase, potyvirus coat protein, CMV coat protein, TMV coat protein, luteovirus replicase, MDMV messenger RNA, mutant geminiviral replicase, *Umbellularia californica* C12:0 preferring acyl-ACP thioesterase, plant C10 or C12:0 preferring acyl-ACP thioesterase, C14:0 preferring acyl-ACP thioesterase (luxD), plant synthase factor A, plant synthase factor B, 6-desaturase, protein having an enzymatic activity in the peroxysomal -oxidation of fatty acids in plant cells, acyl-CoA oxidase, 3-ketoacyl-CoA thiolase, lipase, maize acetyl-CoA-carboxylase, 5-enolpyruvylshikimate-3-phosphate synthase (EPSP), phosphinothricin acetyl transferase (BAR, PAT), CP4 protein, ACC deaminase, ribozyme, protein having posttranslational cleavage site, protein fusion consisting of a DNA-binding domain of Gal4 transcriptional activator and a transcriptional activation domain, a translational fusion of oleosin protein with protein of interest capable of targeting the fusion protein into the lipid phase, DHPS gene conferring sulfonamide resistance, bacterial nitrilase, 2,4-D monooxygenase, acetolactate synthase or acetohydroxyacid synthase (ALS, AHAS), polygalacturonase, bacterial nitrilase, fusion of amino terminal hydrophobic region of a mature phosphate translocator protein residing in the inner envelope membrane of the plastid with protein of interest to be targeted into said membrane etc.
Any human or animal protein can be expressed using the system of the invention. Examples of such proteins of interest include inter alia the following proteins (pharmaceutical proteins): immune response proteins (monoclonal antibodies, single chain antibodies, T cell receptors etc.), antigens, colony stimulating factors, relaxins, polypeptide hormones, cytokines and their receptors, interferons, growth factors and coagulation factors, enzymatically active lysosomal enzyme, fibrinolytic polypeptides, blood clotting factors, trypsinogen, 1-antitrypsin (AAT), as well as function-conservative proteins like fusions, mutant versions and synthetic derivatives of the above proteins.

### EXAMPLES

### EXAMPLE 1

### PVX constructs lacking CP do not move from cell-to-cell

A PVX construct containing a GFP gene (pA3151) was obtained from Professor Yuri Dorokhov (Moscow State University, Russia). This construct was made by cloning a Nhel-Sall fragment containing the GFP coding sequence into pPVX201 (Baulcombe, D., Chapman, S. & Santa Cruz, S., 1995, Plant J., 7:1045-1053) digested with Nhel and Sall, and then subcloning from the resulting construct a Hind3-Sacl fragment (containing the entire viral insert and GFP gene) into pBIN19. The viral insert was then subcloned from pA3151 into plCBV52 (a small pBIN19-based Kan^{R} binary vector) as a Sacl-Sphl fragment, resulting in plasmid plCH20799. This construct contains the PVX construct cloned under the control of the 35S promoter (Fig 1 B). The GFP coding sequence is expressed from a duplicated CP subgenomic promoter (sgc), and is located between the triple gene block (TGB) and the CP coding sequence. Since the duplicated subgenomic promoter fragment upstream of GFP also contained the CP start codon, the start codon was eliminated by mutation from ATG to AGG.

A control construct lacking CP was made from plCH20799 (Fig. 1 B) by deleting the CP gene and its subgenomic promoter (using PCR with one of the primers overlapping the deletion endpoints). The resulting construct, plCH21333 (Fig. 1 B), was transformed into *Agrobacterium* and infiltrated in a *Nicotiana benthamiana* leaf. As expected, viral replicons were unable to move from cell-to-cell or systemically. Movement of the replicons was rescued when plCH21333 was coinfiltrated together with constructs plCH10745 or plCH22066, which provide transient expression of TVCV MP or PVX CP, respectively (Fig. 1A). plCH10745 and plCH22066 constructs contain the TVCV MP or PVX CP coding sequences, respectively, under control of the 35S promoter.

### EXAMPLE 2

### A PVX replicon with the TVCV MP gene cloned between the TGB and the gene of interest is able to move from cell to cell

Since TVCV MP is able to provide cell-to-cell movement to PVX replicons that lack PVX CP, the TVCV MP gene was cloned in the PVX construct between the triple gene block and the gene of interest (GFP in this case), under control of a duplicated CP subgenomic promoter. For practical reasons, this construct (plCH22939, Fig. 2B and SEQ ID NO:1) was made as a 5' provector module which contains the 35S promoter fused to 5' viral vector sequences but lacks the 3' part of the construct including the coding sequence of the gene of interest and the PVX 3' NTR. A recombination site (the Streptomyces phage C31 AttP site) and intron sequences follow the viral construct sequences. A 3' provector module, plCH21282 Fig. 2B), contains a compatible recombination site (Streptomyces phage C31 AttB recombination site) followed by intron sequences, the GFP gene and the PVX NTR. The 5' and 3' parts of the construct are assembled *in vivo* by site-specific recombination at the recombination sites by coinfiltration of agrobacteria containing the 5' and 3' modules and the PhiC31 recombinase (plCH10881, containing the PhiC31 recombinase under control of the Arabidopsis ACT2 promoter, see Marillonnet et al., 2005, Proc. Natl. Acad. Sci. USA, 101: 6852-6857). After T-DNA delivery of the 5' and 3' modules to plant cells and recombination at the AttP and AttB sites, the recombined DNA is transcribed from the 35S promoter. The recombination site is excised from the transcript by splicing of the flanking intron sequences, and the spliced transcript exported from the nucleus to the cytoplasm where it is amplified as a normal viral RNA replicon.
Infiltration of a control provector construct (plCH22922, 5' provector without TVCV MP sequences) with the 3' provector plCH21282 led to the formation of replicons incapable of cell-to-cell movement. In contrast, infiltration of plCH22939 led to replicons that were capable to move from cell to cell (Fig. 2A).
Since TMV is able to provide cell-to-cell movement to TMV replicons without the need for other viral proteins, a second PVX construct was made in which the 12K and 8K proteins of the triple-gene block were deleted, resulting in construct plCH22953 (Fig. 2B and SEQ ID NO:2). In this case, the TMV MP is expressed from the 12K subgenomic promoter (sg12). Infiltration of plCH22953 with plCH21282 led to replicons that were able to move from cell-to-cell. Cell-to-cell movement and GFP fluorescence were however not better or stronger than when using construct plCH22939 (Fig. 2).

### EXAMPLE 3

### A PVX vector with a PVX CP gene cloned between the TGB and the gene of interest is able to move from cell to cell

A construct similar to plCH22939 but with PVX CP replacing TVCV MP was made. The sequence of this construct is the same as that of the wild type virus from the 5' end of the virus up to the end of the CP. The CP is then followed by the duplicated CP subgenomic promoter that is used for expression of the gene of interest. Infiltration of this construct, plCH22988 (Fig. 3B), with plCH21282 (Fig. 2B) led to the formation of replicons that moved from cell to cell much faster and that produced brighter GFP fluorescence than replicons produced from plCH22939 (Fig. 3A).

### EXAMPLE 4

### A PVX vector with a PVX CP gene cloned between the RdRP and the TGB is able to move cell-to-cell

The PVX CP gene was cloned between the RdRp and the triple gene block, under control of a duplicated 25K subgenomic promoter (sg25), resulting in construct plCH24180 (Fig. 4B, SEQ ID NO:3). Infiltration of plCH24180 in combination with plCH21282 and an integrase source (plCH10881) led to the formation of viral replicons that were able to move from cell to cell (Fig. 4A). The construct plCH22577 (similar to plCH22922 but differ for a few restriction sites) was infiltrated instead of plCH24180 in control experiment and exhibited no cell-to-cell movement, as expected. As with plCH22988, GFP fluorescence from plCH24180 was stronger than with construct plCH22939.

### EXAMPLE 5

### Complete assembled PVX viral vectors containing CP also provide cell-to-cell movement.

Assembled constructs including a 3' provector with GFP part (plCH21282, Fig. 2B) and corresponding to provectors plCH22988 or plCH24180 were made, yielding plasmids plCH25488 and plCH25491, respectively (Fig. 5B). The nucleotide sequences of the T-DNA region of plCH25488 and plCH25491 are given as SEQ ID NO:5 and SEQ ID NO:6, respectively. Both constructs were found to work like the provectors, as expected, and provided cell-to-cell movement and strong GFP fluorescence (Fig. 5A). GFP fluorescence was also much stronger than with the standard construct (plCH20799) in which the CP gene is located after the gene of interest suggesting expression of more recombinant protein (Fig. 6A). Coomassie-stained protein gels showed that a much higher amount of GFP protein was expressed from plCH25491 and plCH25488 than from plCH20799, and that in contrast, a much higher amount of PVX CP was produced from plCH20799 than from the other two constructs.

### EXAMPLE 6

### PVX cloning vectors

A schematic representation of two types of PVX cloning vectors with CP between the RdRP and the TGB, or between the TGB and gene of interest, are shown. CS: cloning sites.

## Claims

1. Nucleic acid comprising or encoding an RNA replicon comprising, in this order, the following segments:
(i) a nucleic acid sequence encoding a potexvirus RNA-dependent RNA polymerase or a function-conservative variant thereof;
(ii) a nucleic acid sequence encoding one or more proteins required for cell-to-cell movement of said replicon in a plant or in plant tissue;
(iii) a heterologous nucleic acid sequence expressible from said replicon in a plant or in plant tissue.

2. The nucleic acid according to claim 1, wherein said sequence of item (i) encodes a protein having a sequence identity of at least 36 % to a protein encoded by SEQ ID NO:4 within a protein sequence segment of at least 300 amino acid residues, preferably at least 500 amino acid residues, more preferably at least 900 amino acid residues, and most preferably at least 1400 amino acid residues.

3. The nucleic acid according to claim 1 or 2, wherein said sequence of item (i) encodes a protein having a sequence identity of at least 45 % to a protein encoded by SEQ ID NO:4 within a protein sequence segment of at least 300 amino acid residues, preferably at least 500 amino acid residues, more preferably at least 900 amino acid residues, and most preferably at least 1400 amino acid residues.

4. The nucleic acid according to claim 1, wherein said sequence of item (i) encodes a protein having a sequence homology of at least 50 % to a protein encoded by SEQ ID NO:4 within a protein sequence segment of at least 300 amino acid residues, preferably at least 500 amino acid residues, more preferably at least 900 amino acid residues, most preferably at least 1400 amino acid residues.

5. The nucleic acid according to claim 1, wherein said nucleic acid sequence of item (i) has a sequence identity of at least 55 %, preferably at least 60%, to SEQ ID NO: 4 within a sequence segment of at least 900 nucleotides, preferably at least 1500 nucleotides, more preferably at least 2700 nucleotides, most preferably at least 4200 nucleotides.

6. The nucleic acid according to any one of claims 1 to 5, wherein said at least one nucleic acid sequence of item (ii) comprises a plant viral movement protein (MP).

7. The nucleic acid according to claim 6, wherein said nucleic acid sequence of item (ii) encodes a tobamoviral movement protein (MP), preferably a tobacco mosaic virus MP.

8. The nucleic acid according to any one of claims 1 to 7, wherein said at least one nucleic acid sequence of item (ii) comprises a potexvirus triple gene block or a function-conservative variant thereof.

9. The nucleic acid according to claim 8, wherein said at least one nucleic acid sequence of item (ii) comprises a potexvirus triple gene block or a function-conservative variant thereof and a potexviral coat protein or a function-conservative variant thereof.

10. The nucleic acid according to claim 1 to 9, wherein said replicon is a potexviral replicon, preferably a replicon derived from potato virus X, from bamboo mosaic potexvirus, or from papaya mosaic potexvirus.

11. The nucleic acid according to claim 1 to 10, wherein said replicon does not have an origin of viral particle assembly.

12. The nucleic acid according to any of claims 1 to 11, said nucleic acid comprising a sequence selected from the group: transcription promoter active in plant cells upstream of item (i) of claim 1, viral subgenomic promoter, potexviral 5' or 3' untranslated region.

13. A nucleic acid comprising a complementary sequence of said nucleic acid of any one of claims 1 to 12.

14. Kit of parts comprising at least two vectors that are capable of assembling said nucleic acid of any one of claims 1 to 13 in plant cells by site-directed recombination.

15. The kit of parts according to claim 14, comprising at least two vectors, a first vector comprising or encoding segments of items (i) and (ii) of any one of claims 1 to 13 and a second vector comprising or encoding the segment of item (iii) of any one of claims 1 to 13, wherein said first and said second vector each has a recombination site for allowing assembly of a nucleic acid according to any one of claims 1 to 13 by site-specific recombination.

16. A process of expressing a heterologous nucleic acid sequence of interest in a plant or in plant tissue, comprising providing a plant or plant tissue with said nucleic acid of any one of claims 1 to 13.
